# EUROPEAN PATENT APPLICATION

(11) **EP 3 798 227 A1**
(43) Date of publication of application: **31.03.2021**
(21) Application number: 19799159.9
(22) Date of filing: 09.05.2019
(51) Int. Cl.: C07K 7/08, C07K 16/28, A61K 39/00

(54) **EPITOPE OF REGULATORY T CELL SURFACE ANTIGEN AND ANTIBODY SPECIFICALLY BINDING THERETO**

(30) Priority: 09.05.2018 KR 20180053052
(71) Applicant: Good T Cells, Inc., Seoul 03722 (KR)
(72) Inventor: KIM, Jung Ho, Seoul 04136 (KR); KIM, Beom Seok, Seoul 04029 (KR)
(74) Representative: Schnappauf, Georg
(86) International application number: PCT/KR2019/005592
(87) International publication number: WO 2019/216675

(57) **Abstract**

The present invention relates to an epitope of leucine-rich and immunoglobulin-like domains 1 (Lrig-1) protein, which is an antigen present on the surface of regulatory T cells, and an antibody or antigen-binding fragment specifically binding thereto.

## Description

### Technical Field

The present invention relates to an epitope of leucine-rich and immunoglobulin-like domains 1 (Lrig-1) protein, which is an antigen present on the surface of regulatory T cells, and an antibody or antigen-binding fragment specifically binding thereto.

### Background Art

One of the most important traits in all normal individuals is to have the ability to recognize and eliminate non-self antigens, while not detrimentally responding to antigenic substances that make up the self. As such, non-response of the living body to self antigens is called immunologic unresponsiveness or tolerance. Self-tolerance occurs by eliminating lymphocytes that may have specific receptors for self antigens, or by self-inactivation of the ability to respond after contacting self antigens. In a case where a problem arises in inducing or maintaining self-tolerance, an immune response to self antigens occurs, and the disease resulting therefrom is called autoimmune disease.

For the treatment of the autoimmune disease, a concept of suppressor T cells suggesting the possibility of presence of T cells capable of controlling and suppressing the effector function of conventional T cells was introduced and presented for the first time by Gershon in the early 1970s (R. K. Gershon and K. Kondo, Immunology, 1970, 18: 723-37). Since then, studies have been conducted to elucidate biological properties and functions of regulatory T cells in many areas of immunology.

In this connection, it has been reported that the regulatory T cells (Treg cells) play an important role in naturally preventing occurrence of excessive inflammation and immune responses; however, in a case where autoimmune disease and chronic inflammatory disease occur, the function and the number of the regulatory T cells are remarkably decreased. Therefore, in a case of patients with immune and inflammatory diseases, it is important that the regulatory T cells are produced at a normal level, which can be one of the treatments for these diseases.

Until now, studies on genes and proteins which are present specifically in regulatory T cells have been conducted, and it has been presented that substances such as CD25, CTLA4, CD62L, CD38, CD103, GITR, and CD45RB may correspond to marker substances. However, there are no genes and proteins that can target only the regulatory T cells alone.

On the other hand, there are three hypervariable regions called complementarity determining regions (hereinafter referred to as "CDRs") and four framework regions. The CDRs primarily serve to bind to an epitope on an antigen. The CDRs of each chain are typically referred to as CDR1, CDR2, and CDR3 sequentially starting from the N-terminus, and are also distinguished by the chain where particular CDRs are located.

### Technical Problem

An object of the present invention is to provide an epitope of leucine-rich and immunoglobulin-like domains 1 (Lrig-1) protein present on the surface of regulatory T cells (Treg cells).

Another object of the present invention is to provide an antibody or antigen-binding fragment capable of specifically binding to the epitope.

Yet another object of the present invention is to provide a pharmaceutical composition for preventing or treating cancer, comprising the antibody or antigen-binding fragment capable of specifically binding to the epitope.

Still yet another object of the present invention is to provide a method for preventing or treating cancer, using the antibody or antigen-binding fragment capable of specifically binding to the epitope.

However, the technical problem to be achieved by the present invention is not limited to the above-mentioned problems, and other problems which are not mentioned will be clearly understood by those skilled in the art from the following description.

### Solution to Problem

The present invention relates to an epitope of leucine-rich and immunoglobulin-like domains 1 (Lrig-1) protein, or an antibody or antigen-binding fragment which specifically binds to the epitope.

In the present invention, the "Lrig-1 protein" is a transmembrane protein present on the surface of regulatory T cells, and is composed of leucine-rich repeats (LRRs) and three immunoglobulin-like domains on the extracellular or lumen side, a cell transmembrane sequence, and a cytoplasmic tail portion. The LRIG gene family includes LRIG1, LRIG2, and LRIG3, and the amino acids therebetween are highly conserved. The LRIG1 gene is highly expressed in normal skin and can be expressed in basal and hair follicle cells to regulate proliferation of epithelial stem cells. Therefore, the LRIG1 gene plays an important role in maintaining homeostasis of the epidermis, and its absence may develop psoriasis or skin cancer. It has been reported that in a case where chromosome 3p14.3 portion in which LRIG1 is located is cut off, there is a possibility of developing into cancer cells. In fact, it was identified that expression of LRIG1 is greatly decreased in renal cell carcinoma and cutaneous squamous cell carcinoma. Recently, it has been also found that Lrig-1 is expressed in only about 20 to 30% of cancers. On the other hand, for the purpose of the present invention, the Lrig-1 protein may be, but is not limited to, a protein present in humans or mice.

In an example of the present invention, the Lrig-1 protein may be Lrig-1 protein derived from mammals, including primates such as humans and monkeys, and rodents such as mice and rats.

In an example of the present invention, the Lrig-1 protein may be human-derived Lrig-1 protein represented by SEQ ID NO: 1, which may be encoded by, but is not limited to, a nucleic acid sequence represented by SEQ ID NO: 2 (see Table 1).

**[Table 1]**

| SEQ ID NO | Sequence information |
|---|---|
| SEQ ID NO: 1 |  60 |
| | |
| SEQ ID NO: 2 | atggcgcggc cggtccgggg agggctcggg gccccgcgcc gctcgccttg ccttctcctt 60 ctctggctgc ttttgcttcg gctggagccg gtgaccgccg cggccggccc gcgggcgccc 120 tgcgcggccg cctgcacttg cgctggggac tcgctggact gcggtgggcg cgggctggct 180 gcgttgcccg gggacctgcc ctcctggacg cggagcctaa acctgagtta caacaaactc 240 tctgagattg accctgctgg ttttgaggac ttgccgaacc tacaggaagt gtacctcaat 300 aataatgagt tgacagcggt accatccctg ggcgctgctt catcacatgt cgtctctctc 360 tttctgcagc acaacaagat tcgcagcgtg gaggggagcc agctgaaggc ctacctttcc 420 ttagaagtgt tagatctgag tttgaacaac atcacggaag tgcggaacac ctgctttcca 480 cacggaccgc ctataaagga gctcaacctg gcaggcaatc ggattggcac cctggagttg 540 ggagcatttg atggtctgtc acggtcgctg ctaactcttc gcctgagcaa aaacaggatc 600 acccagcttc ctgtaagagc attcaagcta cccaggctga cacaactgga cctcaatcgg 660 aacaggattc ggctgataga gggcctcacc ttccaggggc tcaacagctt ggaggtgctg 720 aagcttcagc gaaacaacat cagcaaactg acagatgggg ccttctgggg actgtccaag 780 atgcatgtgc tgcacctgga gtacaacagc ctggtagaag tgaacagcgg ctcgctctac 840 ggcctcacgg ccctgcatca gctccacctc agcaacaatt ccatcgctcg cattcaccgc 900 aagggctgga gcttctgcca gaagctgcat gagttggtcc tgtccttcaa caacctgaca 960 cggctggacg aggagagcct ggccgagctg agcagcctga gtgtcctgcg tctcagccac 1020 aattccatca gccacattgc ggagggtgcc ttcaagggac tcaggagcct gcgagtcttg 1080 gatctggacc ataacgagat ttcgggcaca atagaggaca cgagcggcgc cttctcaggg 1140 ctcgacagcc tcagcaagct gactctgttt ggaaacaaga tcaagtctgt ggctaagaga 1200 gcattctcgg ggctggaagg cctggagcac ctgaaccttg gagggaatgc gatcagatct 1260 gtccagtttg atgcctttgt gaagatgaag aatcttaaag agctccatat cagcagcgac 1320 |
| | agcttcctgt gtgactgcca gctgaagtgg ctgcccccgt ggctaattgg caggatgctg 1380 caggcctttg tgacagccac ctgtgcccac ccagaatcac tgaagggtca gagcattttc 1440 tctgtgccac cagagagttt cgtgtgcgat gacttcctga agccacagat catcacccag 1500 ccagaaacca ccatggctat ggtgggcaag gacatccggt ttacatgctc agcagccagc 1560 agcagcagct cccccatgac ctttgcctgg aagaaagaca atgaagtcct gaccaatgca 1620 gacatggaga actttgtcca cgtccacgcg caggacgggg aagtgatgga gtacaccacc 1680 atcctgcacc tccgtcaggt cactttcggg cacgagggcc gctaccaatg tgtcatcacc 1740 aaccactttg gctccaccta ttcacataag gccaggctca ccgtgaatgt gttgccatca 1800 ttcaccaaaa cgccccacga cataaccatc cggaccacca ccatggcccg cctcgaatgt 1860 gctgccacag gtcacccaaa ccctcagatt gcctggcaga aggatggagg cacggatttc 1920 cccgctgccc gtgagcgacg catgcatgtc atgccggatg acgacgtgtt tttcatcact 1980 gatgtgaaaa tagatgacgc aggggtttac agctgtactg ctcagaactc agccggttct 2040 atttcagcta atgccaccct gactgtccta gagaccccat ccttggtggt ccccttggaa 2100 gaccgtgtgg tatctgtggg agaaacagtg gccctccaat gcaaagccac ggggaaccct 2160 ccgccccgca tcacctggtt caagggggac cgcccgctga gcctcactga gcggcaccac 2220 ttgacccctg acaaccagct cctggtggtt cagaacgtgg tggcagagga tgcgggccga 2280 tatacctgtg agatgtccaa caccctgggc acggagcgag ctcacagcca gctgagcgtc 2340 ctgcccgcag caggctgcag gaaggatggg accacggtag gcatcttcac cattgctgtc 2400 gtgagcagca tcgtcctgac gtcactggtc tgggtgtgca tcatctacca gaccaggaag 2460 aagagtgaag agtacagtgt caccaacaca gatgaaaccg tcgtgccacc agatgttcca 2520 agctacctct cttctcaggg gaccctttct gaccgacaag aaaccgtggt caggaccgag 2580 ggtggccctc aggccaatgg gcacattgag agcaatggtg tgtgtccaag agatgcaagc 2640 cactttccag agcccgacac tcacagcgtt gcctgcaggc agccaaagct ctgtgctggg 2700 tctgcgtatc acaaagagcc gtggaaagcg atggagaaag ctgaagggac acctgggcca 2760 cataagatgg aacacggtgg ccgggtcgta tgcagtgact gcaacaccga agtggactgt 2820 tactccaggg gacaagcctt ccacccccag cctgtgtcca gagacagcgc acagccaagt 2880 gcgccaaatg gcccggagcc gggtgggagt gaccaagagc attctccaca tcaccagtgc 2940 agcaggactg ccgctgggtc ctgccccgag tgccaagggt cgctctaccc cagtaaccac 3000 gatagaatgc tgacggctgt gaagaaaaag ccaatggcat ctctagatgg gaaaggggat 3060 tcttcctgga ctttagcaag gttgtatcac ccggactcca cagagctaca gcctgcatct 3120 tcattaactt caggcagtcc agagcgcgcg gaagcccagt acttgcttgt ttccaatggc 3180 cacctcccca aagcatgtga cgccagtccc gagtccacgc cactgacagg acagctcccc 3240 gggaaacaga gggtgccact gctgttggca ccaaaaagct ag |

In another example of the present invention, the Lrig-1 protein may be, but is not limited to, mouse-derived Lrig-1 protein represented by SEQ ID NO: 3 (see Table 2).

**[Table 2]**

| SEQ ID NO | Sequence information |
|---|---|
| SEQ ID NO: 3 | MARPGPGVLG APRLAPRLLL WLLLLLLQWP ESAGAQAGPR APCAAACTCA GDSLDCSGRG 60 LATLPRDLPS WTRSLNLSYN RLSEIDSAAF EDLTNLQEVY LNSNELTAIP SLGTASIGVV 120 SLFLQHNKIL SVDGSQLKSY LSLEVLDLSS NNITEIRSSC FPNGLRIREL NLASNRISIL 180 ESGAFDGLSR SLLTLRLSKN RITQLPVKAF KLPRLTQLDL NRNRIRLIEG LTFQGLDSLE 240 VLRLQRNNIS RLTDGAFWGL SKMHVLHLEY NSLVEVNSGS LYGLTALHQL HLSNNSISRI 300 QRDGWSFCQK LHELILSFNN LTRLDEESLA ELSSLSILRL SHNAISHIAE GAFKGLKSLR 360 VLDLDHNEIS GTIEDTSGAF TGLDNLSKLT LFGNKIKSVA KRAFSGLESL |
| | EHLNLGENAI 420 RSVQFDAFAK MKNLKELYIS SESFLCDCQL KWLPPWLMGR MLQAFVTATC AHPESLKGQS 480 IFSVLPDSFV CDDFPKPQII TQPETTMAVV GKDIRFTCSA ASSSSSPMTF AWKKDNEVLA 540 NADMENFAHV RAQDGEVMEY TTILHLRHVT FGHEGRYQCI ITNHFGSTYS HKARLTVNVL 600 PSFTKIPHDI AIRTGTTARL ECAATGHPNP QIAWQKDGGT DFPAARERRM HVMPDDDVFF 660 ITDVKIDDMG VYSCTAQNSA GSVSANATLT VLETPSLAVP LEDRVVTVGE TVAFQCKATG 720 SPTPRITWLK GGRPLSLTER HHFTPGNQLL VVQNVMIDDA GRYTCEMSNP LGTERAHSQL 780 SILPTPGCRK DGTTVGIFTI AVVCSIVLTS LVWVCIIYQT RKKSEEYSVT NTDETIVPPD 840 VPSYLSSQGT LSDRQETVVR TEGGHQANGH IESNGVCLRD PSLFPEVDIH STTCRQPKLC 900 VGYTREPWKV TEKADRTAAP HTTAHSGSAV CSDCSTDTAY HPQPVPRDSG QPGTASSQEL 960 RQHDREYSPH HPYSGTADGS HTLSGGSLYP SNHDRILPSL KNKAASADGN GDSSWTLAKL 1020 HEADCIDLKP SPTLASGSPE LMEDAISTEA QHLLVSNGHL PKACDSSPES VPLKGQITGK 1080 RRGPLLLAPR S |

According to an embodiment of the present invention, as the epitope of the Lrig-1 protein, there is provided an epitope including a polypeptide that consists of an amino acid sequence represented by Formula 1,

[Formula 1] Lx¹Lx²X³N.

In Formula 1, x¹ to x³ may each independently be a neutral amino acid, an acidic amino acid, a basic amino acid, or an aromatic amino acid. Here, the neutral amino acid may be glycine (G), alanine (A), valine (V), leucine (L), isoleucine (I), serine (S), or threonine (T); the acidic amino acid may be aspartic acid (D), glutamic acid (E), asparagine (N), or glutamine (Q); the basic amino acid may be lysine (K), arginine (R), or histidine (H); and the aromatic amino acid may be phenylalanine (F) or tyrosine (Y).

In an example of the present invention, x¹ to x³ may each independently be an amino acid selected from the group consisting of asparagine (N), aspartic acid (D), serine (S), tyrosine (Y), arginine (R), phenylalanine (F), lysine (K), histidine (H), leucine (L), valine (V), threonine (T), alanine (A), glutamine (Q), glutamic acid (E), and glycine (G).

In another example of the present invention, x¹ may be an amino acid selected from the group consisting of asparagine (N), phenylalanine (F), aspartic acid (D), lysine (K), histidine (H), valine (V), arginine (R), and threonine (T); x² may be an amino acid selected from the group consisting of serine (S), glutamine (Q), alanine (A), asparagine (N), glutamic acid (E), aspartic acid (D), phenylalanine (F), and glycine (G); and x³ may be an amino acid selected from the group consisting of tyrosine (Y), histidine (H), glycine (G), arginine (R), asparagine (N), leucine (L), lysine (K), and phenylalanine (F).

In yet another example of the present invention, x¹ to x³ may each independently be an amino acid selected from the group consisting of asparagine (N), aspartic acid (D), serine (S), tyrosine (Y), and arginine (R).

In still yet another example of the present invention, x¹ may be asparagine (N) or aspartic acid (D); x² may be serine (S) or asparagine (N); and x³ may be tyrosine (Y) or arginine (R).

In the present invention, the epitope includes a polypeptide consisting of an amino acid sequence represented by Formula 1, and may consist of 10- to 20-mer, preferably 10- to 15-mer, and more preferably 11- to 14-mer.

In addition, in the present invention, the polypeptide consisting of the amino acid sequence represented by Formula 1 in the epitope may be located at positions 3 to 6, preferably positions 4 to 6, and more preferably position 5 from the N-terminus of the epitope.

As an example of the present invention, the polypeptide consisting of the amino acid sequence represented by Formula 1 may be represented by, but is not limited to, any one amino acid sequence of SEQ ID NOs: 4 to 17 in Table 3 below:

**[Table 3]**

| SEQ ID NO | Sequence information |
|---|---|
| SEQ ID NO: 4 | LNLSYN |
| SEQ ID NO: 5 | LDLNRN |
| SEQ ID NO: 6 | LFLQHN |
| SEQ ID NO: 7 | LNLAGN |
| SEQ ID NO: 8 | LDLSLN |
| SEQ ID NO: 9 | LRLSKN |
| SEQ ID NO: 10 | LKLQRN |
| SEQ ID NO: 11 | LHLEYN |
| SEQ ID NO: 12 | LHLSNN |
| SEQ ID NO: 13 | LVLSFN |
| SEQ ID NO: 14 | LRLSHN |
| SEQ ID NO: 15 | LDLDHN |
| SEQ ID NO: 16 | LTLFGN |
| SEQ ID NO: 17 | LNLGGN |

As another example of the present invention, the epitope may be represented by, but is not limited to, an amino acid sequence of SEQ ID NO: 18 or 20.

According to another embodiment of the present invention, as the epitope of the Lrig-1 protein, there is provided an epitope including a polypeptide represented by any one amino acid sequence of SEQ ID NOs: 18 to 29 in Table 4 below:

**[Table 4]**

| SEQ ID NO | Sequence information |
|---|---|
| SEQ ID NO: 18 | WTRSLNLSYNKL |
| SEQ ID NO: 19 | TEVRNTCFPHGPPI |
| SEQ ID NO: 20 | RLTQLDLNRNRIR |
| SEQ ID NO: 21 | DLNRNRIRLIEGLTF |
| SEQ ID NO: 22 | NSIARIHRKGW |
| SEQ ID NO: 23 | WLPPWLIGRMLQAF |
| SEQ ID NO: 24 | RQVTFGHEGRY |
| SEQ ID NO: 25 | FGHEGRYQCVITNHF |
| SEQ ID NO: 26 | RLTVNVLPSFTKTHP |
| SEQ ID NO: 27 | RRMHVMPDDDVFF |
| SEQ ID NO: 28 | FFITDVKIDDAGVYS |
| SEQ ID NO: 29 | KGDRPLSLTERHH |

According to yet another embodiment of the present invention, there is provided a nucleic acid molecule encoding the epitope provided by the present invention.

The nucleic acid molecule of the present invention includes all nucleic acid molecules obtained by translating the amino acid sequences of the polypeptides provided by the present invention to polynucleotide sequences, as known to those skilled in the art. Therefore, various polynucleotide sequences may be prepared by an open reading frame (ORF), and all of these polynucleotide sequences are also included in the nucleic acid molecule of the present invention.

According to still yet another embodiment of the present invention, there is provided an expression vector into which the isolated nucleic acid molecule provided by the present invention is inserted.

In the present invention, the "vector" is a nucleic acid molecule capable of transporting another nucleic acid linked thereto. One type of vector is a "plasmid," which refers to circular double-stranded DNA into which an additional DNA segment can be ligated. Another type of vector is a phage vector. Yet another type of vector is a viral vector, where an additional DNA segment can be ligated into the viral genome. Certain vectors are capable of autonomous replication in a host cell into which they are introduced (for example, bacterial vectors having a bacterial origin of replication are episomal mammalian vectors). Other vectors (for example, non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, "plasmid" and "vector" may be used interchangeably as the plasmid is the most commonly used form of vector.

Specific examples of the expression vector in the present invention may be selected from, but are not limited to, the group consisting of commercially widely used pCDNA vectors, F, R1, RP1, Col, pBR322, ToL, Ti vectors; cosmids; phages such as lambda, lambdoid, M13, Mu, p1 P22, Qµµ, T-even, T2, T3, T7; plant viruses. Any expression vector known, to those skilled in the art, as expression vectors can be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation. However, the present invention is not limited thereto, and those skilled in the art may adopt and use an introduction method appropriate for the expression vector and the host cell which are used. The vector may preferably contain at least one selection marker. However, the present invention is not limited thereto, and selection can be made using the vector that contains no selection marker, depending on whether or not a product is produced. The selection marker is selected depending on the target host cell, which is done using methods already known to those skilled in the art, and thus the present invention has no limitation thereon.

In order to facilitate purification of the nucleic acid molecule of the present invention, a tag sequence may be inserted into and fused to an expression vector. The tag includes, but is not limited to, hexa-histidine tag, hemagglutinin tag, myc tag, or flag tag, and any tag known to those skilled in the art which facilitates purification can be used in the present invention.

According to still yet another embodiment of the present invention, there is provided a host cell line, transfected with the expression vector provided by the present invention.

In the present invention, the "host cell" includes individual cells or cell cultures which may be or have been recipients of the vector(s) for incorporation of a polypeptide insert. The host cell includes progeny of a single host cell, and the progeny may not necessarily be completely identical (in morphology or in genomic DNA complement) to the original parent cell due to natural, accidental, or intentional mutation. The host cell includes cells transfected *in vivo* with the polynucleotide(s) herein.

In the present invention, the host cell may include cells of mammalian, plant, insect, fungal, or cellular origin, and may be, for example, bacterial cells such as E. coli, Streptomyces, Salmonella typhimurium; fungal cells such as yeast cells and Pichia pastoris; insect cells such as Drosophila and Spodoptera Sf9 cells; animal cells such as CHO(Chinese hamster ovary cells), SP2/0 (mouse myeloma), human lymphoblastoid, COS, NSO (mouse myeloma), 293T, Bowes melanoma cells, HT-1080, BHK(baby hamster kidney cells), HEK(human embryonic kidney cells), or PERC.6 (human retinal cells); or plant cells. However, the host cell is not limited thereto, and any cell known to those skilled in the art which can be used as a host cell line is available.

According to still yet another embodiment of the present invention, there is provided an antibody or antigen-binding fragment which specifically binds to the epitope of the present invention.

In addition, the antibody according to the present invention specifically binds to an epitope including a polypeptide consisting of an amino acid sequence represented by Formula 1, or an epitope including a polypeptide represented by any one amino acid sequence of SEQ ID NOs: 18 to 29, in Lrig-1 protein present on regulatory T cells, so that the regulatory T cells' function can be suppressed and effector T cells' activity can be maintained or increased, thereby effectively suppressing growth of cancer cells, in particular, solid cancer cells.

As used herein, the "cancer" refers to or indicates a physiological condition characterized by cell growth in mammals which is not regulated in a typical manner. The cancer to be prevented, ameliorated, or treated in the present invention may be solid tumor formed of agglomerates caused by abnormal growth of cells in a solid organ, and may be, but is not limited to, gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, lung cancer, or the like, depending on location of the solid organ.

In the present invention, the antibody as a full-length antibody or as a part of the antibody has the ability to bind to the Lrig-1 protein, and includes any antibody fragment that binds to the Lrig-1 antigen determining site in a competitive manner with the binding molecule of the present invention.

As used herein, the "antibody" refers to a protein molecule which serves as a receptor that specifically recognizes an antigen, including an immunoglobulin molecule that is immunologically reactive with a particular antigen. For the purpose of the present invention, the antigen may be Lrig-1 protein present on the surface of regulatory T cells. Preferably, the antibody may specifically recognize the leucine-rich region or immunoglobulin-like domain of the Lrig-1 protein, but is not limited thereto.

In the present invention, the "immunoglobulin" has a heavy chain and a light chain, and each of the heavy chain and the light chain comprises a constant region and a variable region. The variable region of each of the light chain and the heavy chain contains three hypervariable regions called complementarity determining regions (hereinafter referred to as "CDRs") and four framework regions. The CDRs primarily serve to bind to an epitope on an antigen. The CDRs of each chain are typically referred to as CDR1, CDR2, and CDR3 sequentially starting from the N-terminus, and are also distinguished by the chain where particular CDRs are located.

In the present invention, the "full-length antibody" has a structure with two full-length light chains and two full-length heavy chains in which each light chain is linked to a heavy chain by disulfide bond, and includes IgA, IgD, IgE, IgM, and IgG. The IgG includes, as subtypes thereof, IgG1, IgG2, IgG3, and IgG4.

In addition, as used herein, the "antigen-binding fragment" refers to a fragment having an antigen-binding function, and examples of the antigen-binding fragment include (i) a Fab fragment consisting of a light chain variable region (VL), a heavy chain variable region (VH), a light chain constant region (CL), and a heavy chain constant region 1 (CH1); (ii) a Fd fragment consisting of VH and CH1 domains; (iii) a Fv fragment consisting of VL and VH domains of a single arm of an antibody; (iv) a dAb fragment (Ward, E. S. et al., Nature 341: 544-546 (1989)) consisting of a VH domain; (v) an isolated CDR region; (vi) a F(ab')₂ fragment, which is a bivalent fragment including two linked Fab fragments; (vii) a single-chain Fv molecule (scFv), in which a VH domain and a VL domain are linked by a peptide linker that allows the two domains to associate to form an antigen binding site; (viii) a bispecific single-chain Fv dimer; and (ix) a diabody, which is a multivalent or multispecific fragment constructed by gene fusion. The antigen-binding fragment may be obtained as a Fab or F(ab')₂ fragment in a case where a proteolytic enzyme, for example, papain or pepsin is used, and may be produced through a genetic recombinant technique.

In addition, in the present invention, the antibody may be, but is not limited to, a monoclonal antibody, a polyclonal antibody, a chimeric antibody, a humanized antibody, a bivalent, bispecific molecule, a minibody, a domain antibody, a bispecific antibody, an antibody mimetic, a diabody, a triabody, or a tetrabody, or a fragment thereof.

In addition, as used herein, the "monoclonal antibody" refers to an antibody molecule of a single molecular composition which is obtained from substantially the same antibody population, and exhibits single binding specificity and affinity for a particular epitope.

In the present invention, the "chimeric antibody" is an antibody which is obtained by recombination of a variable region of a mouse antibody and a constant region of a human antibody, and has a greatly improved immune response as compared with the mouse antibody.

In addition, as used herein, the "humanized antibody" refers to an antibody obtained by modifying a protein sequence of an antibody derived from a non-human species so that the protein sequence is similar to an antibody variant naturally produced in humans. For example, the humanized antibody may be prepared as follows. Mouse-derived CDRs may be recombined with a human antibody-derived FR to prepare a humanized variable region, and the humanized variable region may be recombined with a constant region of a preferred human antibody to prepare a humanized antibody.

As used herein, the "binding" or "specific binding" refers to affinity of the antibody or antibody composition herein for an antigen. In antigen-antibody binding, the "specific binding" is distinguishable from non-specific background binding, typically in a case where a dissociation constant (Kd) is less than 1×10⁻⁵M, less than 1×10⁻⁶M, or less than 1×10⁻⁷M. Specific binding can be detected by methods known in the art, such as ELISA, surface plasmon resonance (SPR), immunoprecipitation, and coprecipitation, which include an appropriate control that can distinguish between non-specific binding and specific binding.

The antibody or antigen-binding fragment of the present invention may exist as a multimer, such as a dimer, a trimer, a tetramer, or a pentamer, which includes at least part of antigen-binding capacity of a monomer. Such a multimer also includes a homomultimer or a heteromultimer. Antibody multimers contain a large number of antigen-binding sites, and thus have superior antigen-binding capacity as compared with monomers. Antibody multimers are also easily used to produce multifunctional (that is, bifunctional, trifunctional, tetrafunctional, or the like) antibodies.

As used herein, the "multifunctional" refers to an antibody or antigen-binding fragment which has two or more activities or functions (for example, antigen-binding capacity, enzyme activity, and ligand- or receptor-binding capacity). For example, the antibody of the present invention may be bound to a polypeptide having enzymatic activity, such as luciferase, acetyltransferase, and galactosidase, and the like. Multifunctional antibodies also include multivalent or multispecific (that is, bispecific, trispecific, or the like) forms of antibodies.

According to still yet another embodiment of the present invention, there is provided an antibody-drug conjugate (ADC) comprising the antibody or antigen-binding fragment provided by the present invention and a drug.

As used herein, the "antibody-drug conjugate (ADC)" refers to a form in which the drug and the antibody are chemically linked to each other without degrading biological activity of the antibody and the drug. In the present invention, the antibody-drug conjugate denotes a form in which the drug is bound to an amino acid residue at the N-terminus of the heavy and/or light chain of the antibody, specifically, a form in which the drug is bound to an α-amine group at the N-terminus of the heavy and/or light chain of the antibody.

As used herein, the "drug" may mean any substance having a certain biological activity for a cell, which is a concept including DNA, RNA, or a peptide. The drug may be in a form which contains a reactive group capable of reacting and crosslinking with an α-amine group, and also includes a form which contains a reactive group capable of reacting and crosslinking with an α-amine group and to which a linker is linked.

In the present invention, examples of the reactive group capable of reacting and crosslinking with the α-amine group are not particularly limited in terms of type as long as the reactive group can react and crosslink with an α-amine group at the N-terminus of a heavy or light chain of an antibody. The reactive group includes all types of groups known in the art which react with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, but is not limited thereto.

In the present invention, the antibody-drug conjugate includes, as the antibody or antigen-binding fragment, an antibody or antigen-binding fragment which specifically binds to the epitope of the present invention, that is, an epitope including a polypeptide consisting of an amino acid sequence represented by Formula 1, or an epitope including a polypeptide represented by any one amino acid sequence of SEQ ID NOs: 18 to 29, in Lrig-1 protein, in which the drug may be a drug that can treat cancer, a disease targeted by a Lrig-1 antibody, that is, an anticancer agent.

In the present invention, the anticancer agent may include any drug without limitation as long as the drug is used for prevention, amelioration, or treatment of cancer. The anticancer agent may be, for example, selected from the group consisting of nitrogen mustard, imatinib, oxaliplatin, rituximab, erlotinib, neratinib, lapatinib, gefitinib, vandetanib, nilotinib, semaxanib, bosutinib, axitinib, cediranib, lestaurtinib, trastuzumab, gefitinib, bortezomib, sunitinib, carboplatin, sorafenib, bevacizumab, cisplatin, cetuximab, Viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracil, oteracil, azacitidine, methotrexate, uracil, cytarabine, fluorouracil, fludarabine, enocitabine, flutamide, capecitabine, decitabine, mercaptopurine, thioguanine, cladribine, carmofur, raltitrexed, docetaxel, paclitaxel, irinotecan, belotecan, topotecan, vinorelbine, etoposide, vinblastine, idarubicin, mitomycin, bleomycin, dactinomycin, pirarubicin, aclarubicin, peplomycin, temsirolimus, temozolomide, busulfan, ifosfamide, cyclophosphamide, melphalan, altretamine, dacarbazine, thiotepa, nimustine, chlorambucil, mitolactol, leucovorin, tretinoin, exemestane, aminogluthetimide, anagrelide, olaparib, navelbine, fadrozole, tamoxifen, toremifene, testolactone, anastrozole, letrozole, vorozole, bicalutamide, lomustine, 5FU, vorinostat, entinostat, and carmustine. However, the anticancer agent is not limited thereto.

In the present invention, the cancer may be solid tumor formed of agglomerates caused by abnormal growth of cells in a solid organ, and specific examples thereof may include, but are not limited to, gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, lung cancer, or the like, depending on location of the solid organ.

According to still yet another embodiment of the present invention, there is provided a pharmaceutical composition for preventing or treating cancer, comprising, as an active ingredient, the antibody or antigen-binding fragment provided by the present invention, or the antibody-drug conjugate (ADC) provided by the present invention.

In the present invention, the antibody or antigen-binding fragment, or the antibody-drug conjugate obtained by binding a drug thereto, which is contained as an active ingredient in the pharmaceutical composition, specifically binds to an epitope including a polypeptide consisting of an amino acid sequence represented by Formula 1, or an epitope including a polypeptide represented by any one amino acid sequence of SEQ ID NOs: 18 to 29, in Lrig-1 protein present on regulatory T cells, so that the regulatory T cells' function can be suppressed and effector T cells' activity can be maintained or increased, thereby effectively suppressing growth of cancer cells, in particular, solid cancer cells.

In the present invention, the cancer may be solid tumor formed of agglomerates caused by abnormal growth of cells in a solid organ, and specific examples thereof may include, but are not limited to, gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, lung cancer, or the like, depending on location of the solid organ.

Meanwhile, in the present invention, the "prevention" may include, without limitation, any act of blocking symptoms of a disease, or suppressing or delaying the symptoms, using the pharmaceutical composition of the present invention.

In addition, in the present invention, the "treatment" may include, without limitation, any act of ameliorating or beneficially altering symptoms of a disease, using the pharmaceutical composition of the present invention.

In the present invention, the pharmaceutical composition may be characterized by being in the form of capsules, tablets, granules, injections, ointments, powders, or beverages, and the pharmaceutical composition may be characterized by being targeted to humans.

In the present invention, the pharmaceutical composition may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present invention may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition of the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, time of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the present invention in any way. The pharmaceutical composition according to the present invention may be formulated in the form of pills, sugar-coated tablets, capsules, liquids, gels, syrups, slurries, or suspensions.

According to still yet another embodiment of the present invention, there is provided a method for preventing or treating cancer, comprising a step of administering, to an individual, the antibody or antigen-binding fragment according to the present invention, or the antibody-drug conjugate (ADC) according to the present invention.

The antibody or antigen-binding fragment of the present invention, and the antibody-drug conjugate of the present invention specifically bind to an epitope including a polypeptide consisting of an amino acid sequence represented by Formula 1, or an epitope including a polypeptide represented by any one amino acid sequence of SEQ ID NOs: 18 to 29, in Lrig-1 protein present on regulatory T cells, so that the regulatory T cells' function can be suppressed and effector T cells' activity can be maintained or increased, thereby effectively suppressing growth of cancer cells, in particular, solid cancer cells.

In the present invention, the "individual" is an individual suspected of developing cancer, and the individual suspected of developing cancer means a mammal, such as humans, mice, and domestic animals, who has developed or is likely to develop the disease in question. However, any individual, who is treatable with the antibody or antibody-drug conjugate of the present invention, is included therein without limitation.

The method of the present invention may comprise administering the antibody or the antibody-drug conjugate in a pharmaceutically effective amount. An appropriate total daily amount used may be determined by an attending physician or veterinarian within the scope of sound medical judgment, and administration may be made once or several times. However, for the purposes of the present invention, a specific therapeutically effective amount for a particular patient is preferably applied differently depending on various factors, including type and degree of reaction to be achieved, the specific composition including whether other agents are used therewith as the case may be, the patient's age, body weight, general health status, sex, and diet, time of administration, route of administration, secretion rate of the composition, duration of treatment, and drugs used simultaneously or in combination with the specific composition, and similar factors well known in the medical field.

Meanwhile, the method for preventing or treating cancer may be, but is not limited to, a combination therapy that further comprises administering a compound or substance having therapeutic activity against one or more cancer diseases.

In the present invention, the "combination" should be understood to represent simultaneous, individual, or sequential administration. In a case where the administration is made in a sequential or individual manner, the second component should be administered at intervals such that beneficial effects of the combination are not lost.

In the present invention, a dosage of the antibody or antibody-drug conjugate may be, but is not limited to, about 0.0001 µg to 500 mg per kg of patient's body weight.

In the present invention, the cancer may be solid tumor formed of agglomerates caused by abnormal growth of cells in a solid organ, and specific examples thereof may include, but are not limited to, gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, lung cancer, or the like, depending on location of the solid organ.

### Advantageous Effects of Invention

The antibody or antigen-binding fragment which specifically binds to an epitope of Lrig-1 protein, according to the present invention, specifically binds to the epitope of the present invention present on regulatory T cells, so that the regulatory T cells' function can be suppressed and effector T cells' activity can be maintained or increased, thereby effectively suppressing growth of cancer cells, in particular, solid cancer cells.

### Brief Description of Drawings

FIG. 1 illustrates a structure of the Lrig-1 protein according to an embodiment of the present invention.
FIG. 2 illustrates a structure of the Lrig-1 protein according to an embodiment of the present invention.
FIG. 3 illustrates an expression level of Lrig-1 mRNA according to an embodiment of the present invention.
FIG. 4 illustrates an expression level of Lrig-1 mRNA according to an embodiment of the present invention.
FIG. 5 illustrates an expression level of Lrig-1 mRNA according to an embodiment of the present invention.
FIG. 6 illustrates expression levels of Lrig-1, Lrig-2, and Lrig-3 mRNAs according to an embodiment of the present invention.
FIG. 7 illustrates results obtained by comparing expression levels of Lrig-1 protein in regulatory T cells and non-regulatory T cells according to an embodiment of the present invention.
FIG. 8 illustrates expression of the Lrig-1 protein on the surface of regulatory T cells according to an embodiment of the present invention.
FIG. 9 illustrates results obtained by analyzing binding capacity of antibodies (A7, C8, E7, G3, A8, B8, D9, and H6) to the Lrig-1 protein in an embodiment of the present invention.
FIG. 10 illustrates results obtained by analyzing the mechanism of regulating Lrig-1 protein-induced Stat3 phosphorylation, in regulatory T cells, of Lrig-1 protein-specific monoclonal antibodies (A7, C8, E7, G3, A8, B8, D9, and H6) in an embodiment of the present invention.
FIG. 11 illustrates an experimental design for cancer therapy using Lrig-1 protein-specific monoclonal antibodies (A8, B8, D9, and H6) in an embodiment of the present invention.
FIG. 12 illustrates cancer therapeutic effects obtained by using Lrig-1 protein-specific monoclonal antibodies (A8, B8, D9, and H6) in an embodiment of the present invention.
FIG. 13 illustrates results obtained by performing epitope mapping of 10 µg/ml of a monoclonal antibody (H6) to the Lrig-1 protein using a microarray in an embodiment of the present invention.
FIG. 14 illustrates results obtained by performing epitope mapping of 100 µg/ml of a monoclonal antibody (H6) to the Lrig-1 protein using a microarray in an embodiment of the present invention.
FIG. 15 illustrates results obtained by performing epitope mapping of a monoclonal antibody (H6) to the Lrig-1 protein using a microarray in an embodiment of the present invention.

### Detailed Description of Invention

The present invention relates to an epitope of leucine-rich and immunoglobulin-like domains 1 (Lrig-1) protein, or an antibody or antigen-binding fragment which specifically binds to the epitope.

According to an embodiment of the present invention, as the epitope of the Lrig-1 protein, there is provided an epitope including a polypeptide that consists of an amino acid sequence represented by Formula 1,

[Formula 1] Lx¹Lx²x³N.

In Formula 1, x¹ to x³ may each independently be a neutral amino acid, an acidic amino acid, a basic amino acid, or an aromatic amino acid. Here, the neutral amino acid may be glycine (G), alanine (A), valine (V), leucine (L), isoleucine (I), serine (S), or threonine (T); the acidic amino acid may be aspartic acid (D), glutamic acid (E), asparagine (N), or glutamine (Q); the basic amino acid may be lysine (K), arginine (R), or histidine (H); and the aromatic amino acid may be phenylalanine (F) or tyrosine (Y).

In an example of the present invention, x¹ to x³ may each independently be an amino acid selected from the group consisting of asparagine (N), aspartic acid (D), serine (S), tyrosine (Y), arginine (R), phenylalanine (F), lysine (K), histidine (H), leucine (L), valine (V), threonine (T), alanine (A), glutamine (Q), glutamic acid (E), and glycine (G).

In another example of the present invention, x¹ may be an amino acid selected from the group consisting of asparagine (N), phenylalanine (F), aspartic acid (D), lysine (K), histidine (H), valine (V), arginine (R), and threonine (T); x² may be an amino acid selected from the group consisting of serine (S), glutamine (Q), alanine (A), asparagine (N), glutamic acid (E), aspartic acid (D), phenylalanine (F), and glycine (G); and x³ may be an amino acid selected from the group consisting of tyrosine (Y), histidine (H), glycine (G), arginine (R), asparagine (N), leucine (L), lysine (K), and phenylalanine (F).

In yet another example of the present invention, x¹ to x³ may each independently be an amino acid selected from the group consisting of asparagine (N), aspartic acid (D), serine (S), tyrosine (Y), and arginine (R).

In still yet another example of the present invention, x¹ may be asparagine (N) or aspartic acid (D); x² may be serine (S) or asparagine (N); and x³ may be tyrosine (Y) or arginine (R).

In still yet another example of the present invention, the polypeptide consisting of the amino acid sequence represented by Formula 1 may be represented by, but is not limited to, any one amino acid sequence of SEQ ID NOs: 4 to 17.

According to another embodiment of the present invention, as the epitope of the Lrig-1 protein, there is provided an epitope including a polypeptide represented by any one amino acid sequence of SEQ ID NOs: 18 to 29 in Table 4.

According to yet another embodiment of the present invention, there is provided an antibody or antigen-binding fragment which specifically binds to the epitope of the present invention.

According to still yet another embodiment of the present invention, there is provided a pharmaceutical composition for preventing or treating cancer, comprising, as an active ingredient, the antibody or antigen-binding fragment provided by the present invention.

Hereinafter, the present invention will be described in more detail by way of examples. These examples are only for describing the present invention in more detail, and it will be apparent to those skilled in the art that according to the gist of the present invention, the scope of the present invention is not limited by these examples.

### Examples

### [Preparation Example 11 T cell subtype cell culture

In order to identify whether the Lrig-1 protein is expressed only in regulatory T cells (Treg) cells, the subsets of T cells, Th0, Th1, Th2, Th17, and iTreg, were prepared. The iTreg refers to cells whose differentiation has been artificially induced in a medium having the following composition, unlike nTreg which has been naturally isolated.

The subsets of the T cells were induced to differentiate into respective cells by first isolating naive T cells obtained from the spleen of mice, causing RPMI1640 (Invitrogen Gibco, Grand Island, NY) nutrient medium that contains 10% fetal bovine serum (FBS; HyClone, Logan, UT) to further contain the respective ingredients of Table 5 below, and performing 72-hour incubation in an incubator at 37°C, 5% CO₂.

**[Table 5]**

| Differentiated cell | Composition |
|---|---|
| Th0 | anti-CD3, anti-CD28 |
| Th1 | IL-12, anti-IL-4 antibody |
| Th2 | IL-4, anti-IFNβ |
| Th17 | IL-6, TGFβ, anti-IFNβ, anti-IL-4 |
| iTreg | IL-2, TGFβ |

### [Example 11 Structural analysis of Lrig-1

A three-dimensional steric structure of the extracellular domain of the Lrig-1 protein was predicted to produce an antibody specific for the Lrig-1 protein, a surface protein of regulatory T cells.

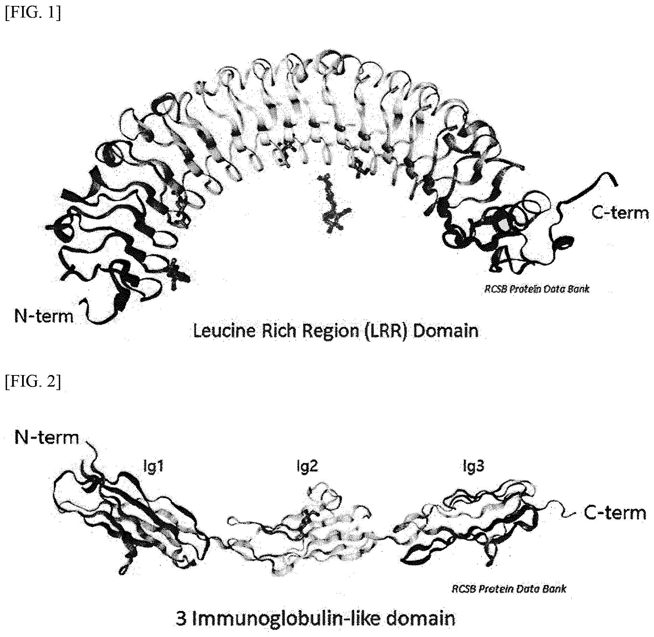

First, in order to predict base sequences of epitopes, tools of Uniprot (http://www.uniprot.org) and RCSB Protein Data Bank (http://www.rcsb.org/pdb) were used to predict a three-dimensional steric structure of the extracellular domain (ECD) of the Lrig-1 protein so that the structure of ECD is identified. Then, the results are illustrated in FIGS. 1 and 2.

As illustrated in FIG. 1, a total of 15 leucine-rich regions of LRR1 to LRR15 exist in the Lrig-LRR domain (amino acid sequence at positions 41 to 494) in the extracellular domain of the Lrig-1 protein. Each of the LRR domains is composed of 23 to 27 amino acids, with 3 to 5 leucine being present.

In addition, as illustrated in FIG. 2, three immunoglobulin-like domains exist in amino acid sequences at positions 494 to 781 of the Lrig-1 protein in the extracellular domain of the Lrig-1 protein.

### [Example 21 Identification of specific expression of Lrig-1 mRNA in regulatory T cells

Verification was made of whether the Lrig-1 protein can act as a biomarker specific for regulatory T cells.

For the verification, CD4⁺ T cells were isolated using magnet-activated cell sorting (MACS), through CD4 beads, from the spleen of mice. Subsequently, regulatory T (CD4⁺CD25⁺T) cells and non-regulatory T (CD4⁺CD25⁻T) cells were isolated with a fluorescence-activated cell sorter (FACS) using a CD25 antibody. For the respective cells and the cells differentiated in Preparation Example 1, mRNA was extracted using Trizol, and then gDNA was removed from genomic RNA using gDNA extraction kit (Qiagen) according to the protocol provided by the manufacturer. The gDNA-removed mRNA was synthesized into cDNA through the BDsprint cDNA Synthesis Kit (Clonetech).

Real-time polymerase chain reaction (RT PCR) was performed to quantitatively identify an expression level of Lrig-1 mRNA in the cDNA.

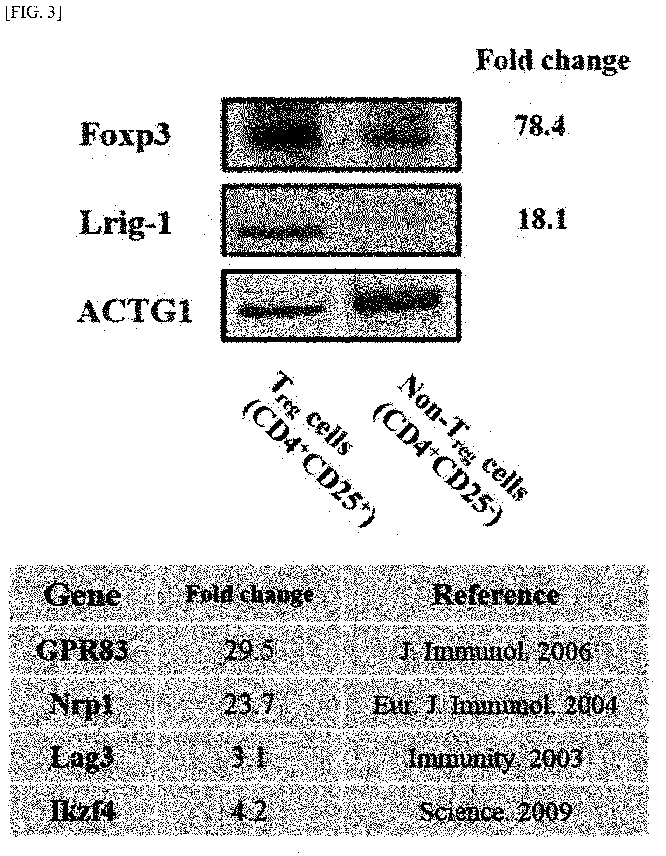

The real-time polymerase chain reaction was performed with primers shown in Table 6 below using SYBR Green (Molecular Probes) according to the protocol provided by the manufacturer under conditions of 40 cycles consisting of 95°C for 3 minutes, 61°C for 15 seconds, 72°C for 30 seconds; and a relative gene expression level was calculated using the ΔCT method, and normalized using HPRT. The results are illustrated in FIGS. 3 to 6.

**[Table 6]**

| Primer | Sequence |
|---|---|
| Mouse Lrig-1 | Forward 5' - GAC GGAATT CAG TGA GGA GAA CCT - 3' |
| | Reverse 5' - CAA CTG GTA GTG GCA GCT TGT AGG - 3' |
| Mouse Lrig-2 | Forward 5' - TCA CAA GGA ACA TTG TCT GAA CCA- 3' |
| | Reverse 5' - GCC TGA TCT AAC ACA TCC TCC TCA- 3' |
| Mouse Lrig-3 | Forward 5' - CAG CAC CTT GAG CTG AAC AGA AAC - 3' |
| | Reverse 5' - CCA GCC TTT GGT AAT CTC GGT TAG - 3' |
| Mouse FOXP3 | Forward 5' - CTT TCA CCT ATC CCA CCC TTA TCC - 3' |
| | Reverse 5' - ATT CAT CTA CGG TCC ACA CTG CTC - 3' |
| ACTG1 | Forward 5' - GGC GTC ATG GTG GGC ATG GG - 3' |
| | Reverse 5' - ATG GCG TGG GGAAGG GCG TA - 3' |

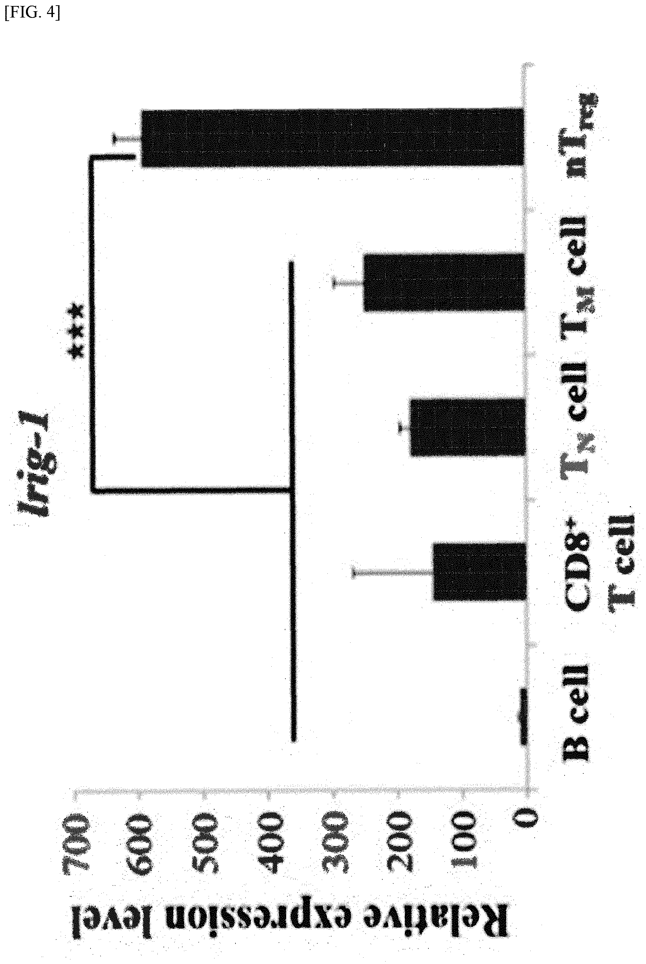

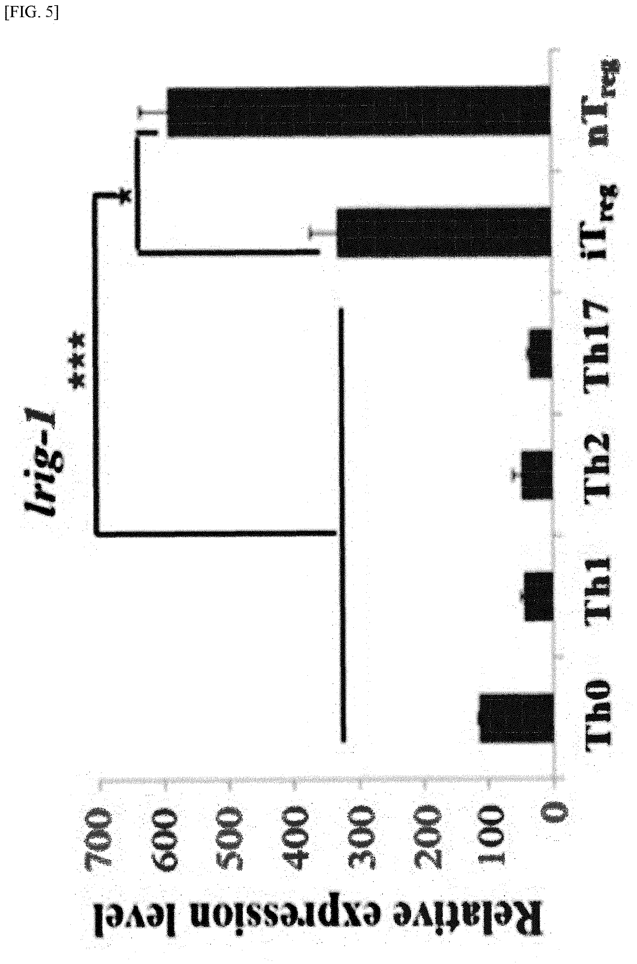

As illustrated in FIG. 3, it can be seen that the expression of Lrig-1 in regulatory T (CD4⁺CD25⁺ T) cells is 18.1 times higher than non-regulatory T (CD4⁺CD25⁻ T) cells. This was about 10 times higher expression level than Lag3 and Ikzf4, which are previously known markers for regulatory T cells. In addition, as illustrated in FIGS. 4 and 5, the expression of Lrig-1 mRNA was remarkably high in regulatory T cells as compared with other types of immune cells, and in particular, was remarkably high in naturally isolated regulatory T cells (nTreg) as compared with induced regulatory T cells (iTreg).

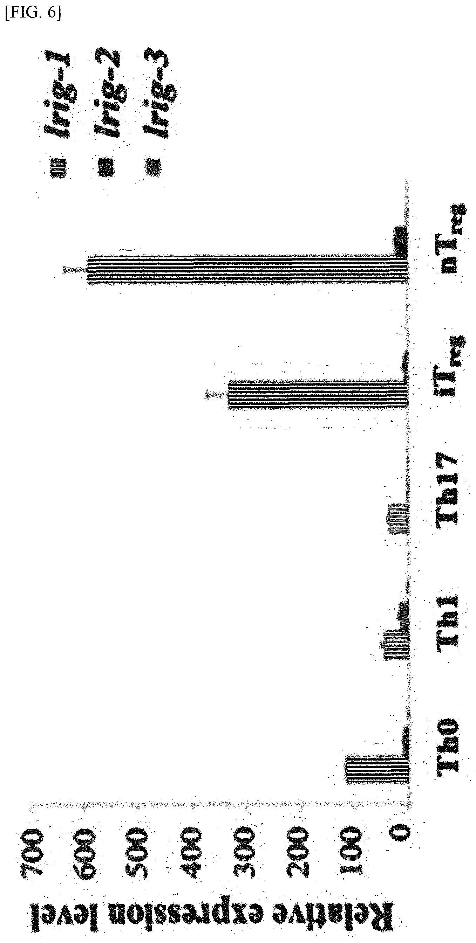

In addition, as illustrated in FIG. 6, the expression of Lrig-1 was the highest among Lrig-1, Lrig-2, and Lrig-3 which correspond to the Lrig family.

From the above results, it can be seen that the Lrig-1 protein according to the present invention is specifically expressed in regulatory T cells, in particular, naturally-occurring regulatory T cells.

### [Example 31 Identification of specific expression of Lrig-1 protein in regulatory T cells

It was identified whether the Lrig-1 protein expressed from Lrig-1 mRNA is specifically expressed only in regulatory T cells.

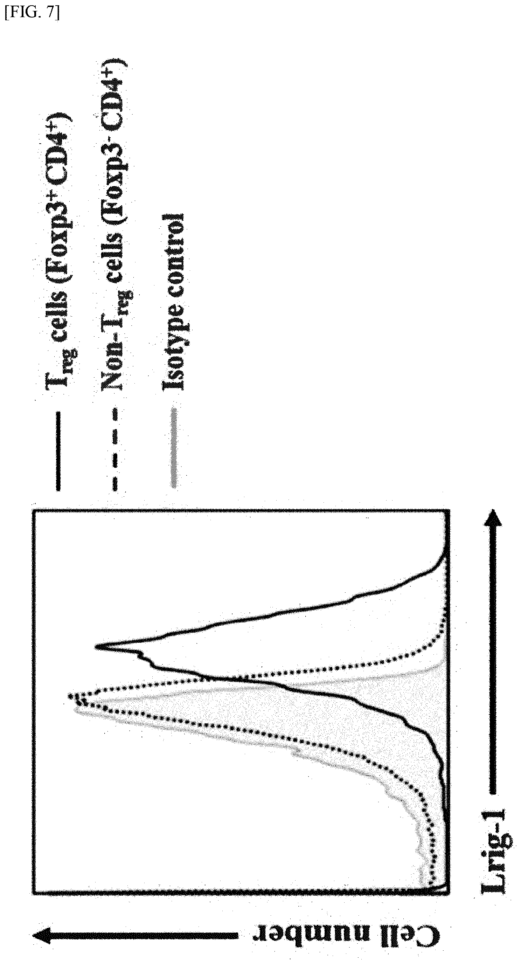

Using FOXP3-RFP-knocked-in mice, the FOXP3-RFP obtained by coupling red fluorescence protein (RFP) to FOXP3 promoter, which is a transcription factor specific for regulatory T cells, CD4⁺ T cells were isolated using magnet-activated cell sorting (MACS), through CD4 beads, from the spleen of the mice. Subsequently, using RFP protein, regulatory T (CD4⁺RFP⁺ T) cells and non-regulatory T (CD4⁺RFP⁻ T) cells were obtained by performing isolation through a fluorescence-activated cell sorter (FACS). The respective cells were stained with the purchased Lrig-1 antibody and a negative control was stained with an isotype-matched control antibody, to measure an expression level of Lrig-1 with the fluorescence-activated cell sorter. The results are illustrated in FIG. 7.

As illustrated in FIG. 7, the non-regulatory T cells indicated by a dotted line showed almost the same expression level of Lrig-1 as the negative control, whereas there were a large number of cells with high expression level of Lrig-1 in the regulatory T cells.

From the above results, it can be seen that the Lrig-1 protein according to the present invention is specifically expressed in regulatory T cells.

### [Example 41 Identification of specific expression of Lrig-1 protein on surface of regulatory T cells

From the viewpoint that in order to be a target of cell therapy, the Lrig-1 protein must be expressed on the surface of regulatory T cells, which in turn allows a more effective target therapy, it was identified whether the Lrig-1 protein is expressed on the surface of the regulatory T cells.

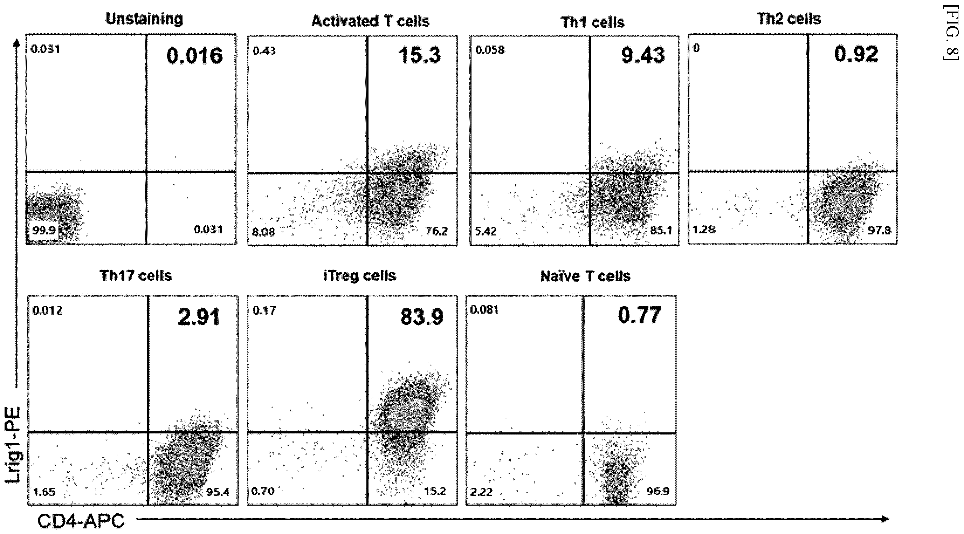

The respective differentiated T cell subsets of Preparation Example 1 were stained with anti-CD4-APC and anti-Lrig-1-PE antibodies, and expression levels of Lrig-1 were measured at the respective cell surfaces using a fluorescence-activated cell sorter (FACS). The results are illustrated in FIG. 8.

As illustrated in FIG. 8, Lrig-1 was expressed in an amount of 0.77 to 15.3 in activated T cells, Th1 cells, Th2 cells, Th17 cells, and naive T cells, whereas Lrig-1 was expressed as high as 83.9 in differentiation-induced T cells (iTreg cells).

From the above results, it can be seen that the Lrig-1 protein according to the present invention is not only specifically expressed in regulatory T (Treg) cells, but also is, in particular, expressed at a higher level on the surface of the Treg cells.

### [Production Examples 1 to 81 Production of monoclonal antibodies specific for Lrig-1 protein

Antibodies specific for the Lrig-1 protein according to the present invention were produced. The present antibodies were not produced by specifying a certain epitope, but were produced as antibodies capable of binding to any site on the Lrig-1 protein.

In order to produce the antibodies, cells expressing the Lrig-1 protein were produced. More specifically, a DNA fragment corresponding to SEQ ID NO: 2 and pcDNA (hygro) were cleaved with a cleavage enzyme, incubated at 37°C, and ligated to produce pcDNA into which a DNA sequence of the Lrig-1 protein is inserted. The thus produced pcDNA into which SEQ ID NO: 2 is inserted was introduced, through transfection, into L cells, so that the Lrig-1 protein is allowed to be expressed on the surface of the L cells.

Light and heavy chain amino acid sequences capable of binding to Lrig-1 expressed on the cell surface were selected from the Human scFv library so that a total of eight heavy and light chains were selected.

The selected heavy and light chain amino acid sequences were fused with the mlgG2a Fc region, to produce monoclonal antibodies. The sequences of the monoclonal antibodies are shown in Table 7 below.

**[Table 7]**

| Classification | Clone | Location | Amino acid sequence | Sequence information |
|---|---|---|---|---|
| Production Example 1 | A7 clone | Heavy chain | EVQLLESGGGLVQPGGSLRLSCAASGFTFSGYDMSWVRQAPGKGLEWVSLIYP DSGNKYYADSVKGRFTISRDNSKNTLYLQMNSLRAEDTAVYYCARDAGLSWA GAFDYWGQGTLVTVSSTTAPSVYPLAPVCGDTTGSSVTLGCLVKGYFPEPVTLT WNSGSLSSGVHTFPAVLQSDLYTLSSSVTVTSSTWPSQSITCNVAHPASSTKVDK KIEPRGPTIKPCPPCKCPAPNLLGGPSVFIFPPKIKDVLMISLSPIVTCVVVDVSED DPDVQISWFVNNVEVHTAQTQTHREDYNSTLRVVSALPIQHQDWMSGKEFKC KVNNKDLPAPIERTISKPKGSVRAPQVYVLPPPEEEMTKKQVTLTCMVTDFMPE DIYVEWTNNGKTELNYKNTEPVLDSDGSYFMYSKLRVEKKNWVERNSYSCSV VHEGLHNHHTTKSFSRTPGK | SEQ ID NO: 30 |
| | | Light chain | QSVLTQPPSASGTPGQRVTISCSGSSSNIGSNYVTWYQQLPGTAPKLLIYSDSHRP SGVPDRFSGSKSGTSASLAISGLQSEDEADYYCGSWDYSLSAYVFGGGTKLTVL RTVAAPTVSIFPPSSEQLTSGGASVVCFLNNFYPKDINVKWKIDGSERQNGVLNS WTDQDSKDSTYSMSSTLTLTKDEYERHNSYTCEATHKTSTSPIVKSFNRNEC | SEQ ID NO: 31 |
| Production Example 2 | C8 clone | Heavy chain | EVQLLESGGGLVQPGGSLRLSCAASGFTFSNYYMSWVRQAPGKGLEWVSGISP GDSSTYYADSVKGRFTISRDNSKNTLYLQMNSLRAEDTAVYYCAKGLYSNPNEP FDYWGQGTLVTVSSTTAPSVYPLAPVCGDTTGSSVTLGCLVKGYFPEPVTLTWN SGSLSSGVHTFPAVLQSDLYTLSSSVTVTSSTWPSQSITCNVAHPASSTKVDKKIE PRGPTIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVN NKDLPAPIERTISKPKGSVRAPQVYVLPPPEEEMTKKQVTLTCMVTDFMPEDIY VEWTNNGKTELNYKNTEPVLDSDGSYFMYSKLRVEKKNWVERNSYSCSVVHE GLHNHHTTKSFSRTPGK | SEQ ID NO: 32 |
| | | Light chain | QSVLTQPPSASGTPGQRVTISCTGSSSNIGSNYVSWYQQLPGTAPKLLIYDDSQR PSGVPDRFSGSKSGTSASLAISGLRSEDEADYYCGTWDYSLNGYVFGGGTKLTV LRTVAAPTVSIFPPSSEQLTSGGASVVCFLNNFYPKDINVKWKIDGSERQNGVLN SWTDQDSKDSTYSMSSTLTLTKDEYERHNSYTCEATHKTSTSPIVKSFNRNEC | SEQ ID NO: 33 |
| Production Example 3 | E7 clone | Heavy chain | EVQLLESGGGLVQPGGSLRLSCAASGFTFSSYDMSWVRQAPGKGLEWVSGISP DGSNIYYADSVKGRFTISRDNSKNTLYLQMNSLRAEDTAVYYCAKVGLRCRYE ACSYAYGMDVWGQGTLVTVSSTTAPSVYPLAPVCGDTTGSSVTLGCLVKGYFP EPVTLTWNSGSLSSGVHTFPAVLQSDLYTLSSSVTVTSSTWPSQSITCNVAHPASS TKVDKKIEPRGPTIKPCPPCKCPAPNLLGGPSVFIFPPKIKDVLMISLSPIVTCVVV DVSEDDPDVQISWFVNNVEVHTAQTQTHREDYNSTLRVVSALPIQHQDWMSG KEFKCKVNNKDLPAPIERTISKPKGSVRAPQVYVLPPPEEEMTKKQVTLTCMVT DFMPEDIYVEWTNNGKTELNYKNTEPVLDSDGSYFMYSKLRVEKKNWVERNS YSCSVVHEGLHNHHTTKSFSRTPGK | SEQ ID NO: 34 |
| | | Light chain | QSVLTQPPSASGTPGQRVTISCSGSSSNIGSNYVSWYQQLPGTAPKLLIYSDSHRP SGVPDRFSGSKSGTSASLAISGLRSEDEADYYCATWDSSLNGYVFGGGTKLTVL RTVAAPTVSIFPPSSEQLTSGGASVVCFLNNFYPKDINVKWKIDGSERQNGVLNS WTDQDSKDSTYSMSSTLTLTKDEYERHNSYTCEATHKTSTSPIVKSFNRNEC | SEQ ID NO: 35 |
| Production Example 4 | G3 clone | Heavy chain | EVQLLESGGGLVQPGGSLRLSCAASGFTFSNYDMSWVRQAPGKGLEWVSSISP SSGSIYYADSVKGRFTISRDNSKNTLYLQMNSLRAEDTAVYYCAKDLDAFWRPS FDYWGQGTLVTVSSTTAPSVYPLAPVCGDTTGSSVTLGCLVKGYFPEPVTLTWN SGSLSSGVHTFPAVLQSDLYTLSSSVTVTSSTWPSQSITCNVAHPASSTKVDKKIE PRGPTIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVN NKDLPAPIERTISKPKGSVRAPQVYVLPPPEEEMTKKQVTLTCMVTDFMPEDIY VEWTNNGKTELNYKNTEPVLDSDGSYFMYSKLRVEKKNWVERNSYSCSVVHE GLHNHHTTKSFSRTPGK | SEQ ID NO: 36 |
| | | Light chain | QSVLTQPPSASGTPGQRVTISCTGSSSNIGNNNVNWYQQLPGTAPKLLIYSDSHR PSGVPDRFSGSKSGTSASLAISGLRSEDEADYYCGSWDDSLSAY VFGGGTKLTV LRTVAAPTVSIFPPSSEQLTSGGASVVCFLNNFYPKDINVKWKIDGSERQNGVLN SWTDQDSKDSTYSMSSTLTLTKDEYERHNSYTCEATHKTSTSPIVKSFNRNEC | SEQ ID NO: 37 |
| Production Example 5 | A8 clone | Heavy chain | EVQLLESGGGLVQPGGSLRLSCAASGFTFSDYDMSWVRQVPGKGLEWVSWIS HGGGSIYYADSVKGRFTISRDNSKNTLYLQMNSLRAEDTAVYYCARGLGLCKT GLCYYYDAMDVWGQGTLVTVSSTTAPSVYPLAPVCGDTTGSSVTLGCLVKGY FPEPVTLTWNSGSLSSGVHTFPAVLQSDLYTLSSSVTVTSSTWPSQSITCNVAHPA SSTKVDKKIEPRGPTIKPCPPCKCPAPNLLGGPSVFIFPPKIKDVLMISLSPIVTCV VVDVSEDDPDVQISWFVNNVEVHTAQTQTHREDYNSTLRVVSALPIQHQDWM SGKEFKCKVNNKDLPAPIERTISKPKGSVRAPQVYVLPPPEEEMTKKQVTLTCM VTDFMPEDIYVEWTNNGKTELNYKNTEPVLDSDGSYFMYSKLRVEKKNWVER NSYSCSVVHEGLHNHHTTKSFSRTPGK | SEQ ID NO: 38 |
| | | Light chain | QSVLTQPPSASGTPGQRVTISCTGSSSNIGNNSVTWYQQLPGTAPKLLIYADNNR PSGVPDRFSGSKSGTSASLAISGLRSEDEADYYCAAWDSSLSAYVFGGGTKLTV LRTVAAPTVSIFPPSSEQLTSGGASVVCFLNNFYPKDINVKWKIDGSERQNGVLN SWTDQDSKDSTYSMSSTLTLTKDEYERHNSYTCEATHKTSTSPIVKSFNRNEC | SEQ ID NO: 39 |
| Production Example 6 | B8 clone | Heavy chain | EVQLLESGGGLVQPGGSLRLSCAASGFTFSDYYMSWVRQAPGKGLEWVSGISH DSGSKYYADSVKGRFTISRDNSKNTLYLQMNSLRAEDTAVYYCARHWTTFDY WGQGTLVTVSSTTAPSVYPLAPVCGDTTGSSVTLGCLVKGYFPEPVTLTWNSGS LSSGVHTFPAVLQSDLYTLSSSVTVTSSTWPSQSITCNVAHPASSTKVDKKIEPRG PTIKPCPPCKCPAPNLLGGPSVFIFPPKIKDVLMISLSPIVTCVVVDVSEDDPDVQI SWFVNNVEVHTAQTQTHREDYNSTLRVVSALPIQHQDWMSGKEFKCKVNNKD LPAPIERTISKPKGSVRAPQVYVLPPPEEEMTKKQVTLTCMVTDFMPEDIYVEW TNNGKTELNYKNTEPVLDSDGSYFMYSKLRVEKKNWVERNSYSCSVVHEGLH NHHTTKSFSRTPGK | SEQ ID NO: 40 |
| | | Light chain | QSVLTQPPSASGTPGQRVTISCSGSSSNIGSNNVTWYQQLPGTAPKLLIYANSNRP SGVPDRFSGSKSGTSASLAISGLRSEDEADYYCGAWDYSLSAYVFGGGTKLTVL RTVAAPTVSIFPPSSEQLTSGGASVVCFLNNFYPKDINVKWKIDGSERQNGVLNS WTDQDSKDSTYSMSSTLTLTKDEYERHNSYTCEATHKTSTSPIVKSFNRNEC | SEQ ID NO: 41 |
| Production Example 7 | D9 clone | Heavy chain | EVQLLESGGGLVQPGGSLRLSCAASGFTFSNYAMSWVRQAPGKGLEWVSAIYP GGGSIYYADSVKGRFTISRDNSKNTLYLQMNSLRAEDTAVYYCARDILPCPWGR CYYDYAMDVWGQGTLVTVSSTTAPSVYPLAPVCGDTTGSSVTLGCLVKGYFPE PVTLTWNSGSLSSGVHTFPAVLQSDLYTLSSSVTVTSSTWPSQSITCNVAHPASST KVDKKIEPRGPTIKPCPPCKCPAPNLLGGPSVFIFPPKIKDVLMISLSPIVTCVVVD VSEDDPDVQISWFVNNVEVHTAQTQTHREDYNSTLRVVSALPIQHQDWMSGK EFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSY SCSVVHEGLHNHHTTKSFSRTPGK | SEQ ID NO: 42 |
| | | Light chain | QSVLTQPPSASGTPGQRVTISCSDSSSNIGSNTVSWYQQLPGTAPKLLIYADNNRP SGVPDRFSGSKSGTSASLAISGLRSEDEADYYCGTWDYSLSGYVFGGGTKLTVL RTVAAPTVSIFPPSSEQLTSGGASVVCFLNNFYPKDINVKWKIDGSERQNGVLNS WTDQDSKDSTYSMSSTLTLTKDEYERHNSYTCEATHKTSTSPIVKSFNRNEC | SEQ ID NO: 43 |
| Production Example 8 | H6 clone | Heavy chain | EVQLLESGGGLVQPGGSLRLSCAASGFTFSNYAMSWVRQAPGKGLEWVSVISH GGGSTYYADSVKGRFTISRDNSKNTLYLQMNSLRAEDTAVYYCARVISNCHLG VCYYSNGMDVWGQGTLVTVSSTTAPSVYPLAPVCGDTTGSSVTLGCLVKGYFP EPVTLTWNSGSLSSGVHTFPAVLQSDLYTLSSSVTVTSSTWPSQSITCNVAHPASS TKVDKKIEPRGPTIKPCPPCKCPAPNLLGGPSVFIFPPKIKDVLMISLSPIVTCVVV DVSEDDPDVQISWFVNNVEVHTAQTQTHREDYNSTLRVVSALPIQHQDWMSG KEFKCKVNNKDLPAPIERTISKPKGSVRAPQVYVLPPPEEEMTKKQVTLTCMVT DFMPEDIYVEWTNNGKTELNYKNTEPVLDSDGSYFMYSKLRVEKKNWVERNS YSCSVVHEGLHNHHTTKSFSRTPGK | SEQ ID NO: 44 |
| | | Light chain | QSVLTQPPSASGTPGQRVTISCSGSSSNIGNNDVYWYQQLPGTAPKLLIYSDSQR PSGVPDRFSGSKSGTSASLAISGLRSEDEADYYCGTWDYSLSGYVFGGGTKLTV LRTVAAPTVSIFPPSSEQLTSGGASVVCFLNNFYPKDINVKWKIDGSERQNGVLN SWTDQDSKDSTYSMSSTLTLTKDEYERHNSYTCEATHKTSTSPIVKSFNRNEC | SEQ ID NO: 45 |

### [Example 51 Evaluation of binding capacity of antibodies according to present invention to Lrig-1 protein

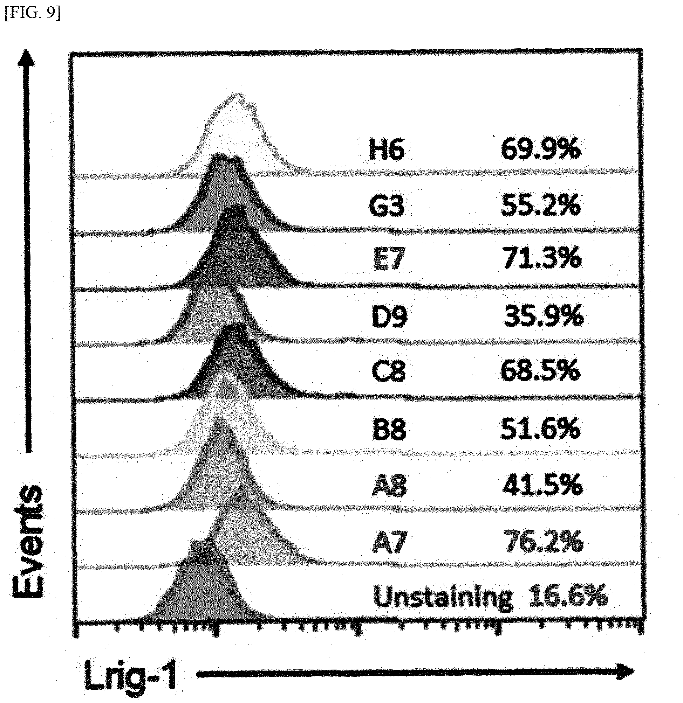

In order to identify whether the monoclonal antibodies according to the present invention produced in Production Examples 1 to 8 well recognize Lrig-1, each of the antibodies of Production Examples 1 to 8 was bound to L cells that stably express Lrig-1. Then, a secondary antibody which is conjugated with eFlour 670 and is capable of recognizing mouse antibodies was added thereto, and then binding capacity of the monoclonal antibodies to the Lrig-1 protein was analyzed using FACS. The results are illustrated in FIG. 9.

As illustrated in FIG. 9, it was found that all Lrig-1 protein-specific monoclonal antibodies (A7, A8, B8, C8, D9, E7, G3, and H6) according to the present invention effectively recognize and bind to the Lrig-1 protein present on the surface of L cells.

### [Example 61 Regulation of signal transduction pathway in regulatory T cells, by antibodies according to present invention

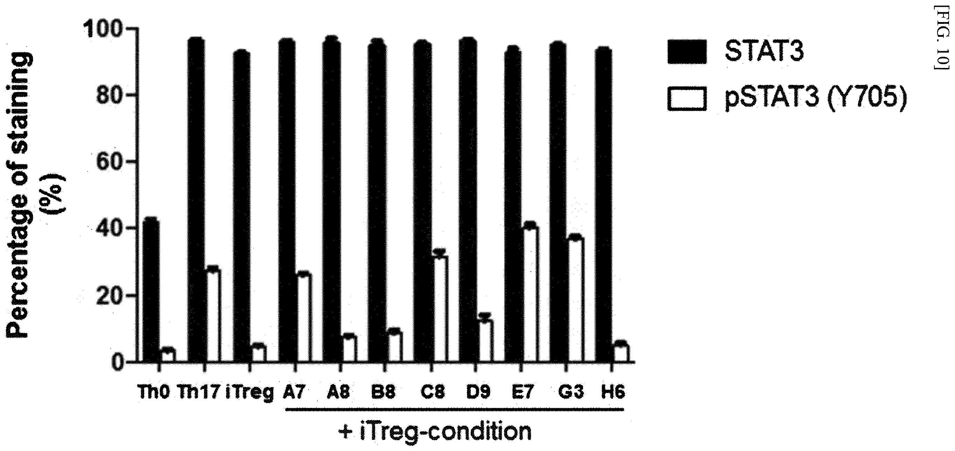

In order to analyze how the monoclonal antibodies according to the present invention produced in Production Examples 1 to 8 affect the signal transduction pathway in regulatory T cells through the Lrig-1 protein, Lrig-1 present on the surface of the regulatory T cells was stimulated by treating the regulatory T cells with the antibodies of Production Examples 1 to 8, and then a level of tyrosine phosphorylation of Stat3 protein present in the stimulated regulatory T cells was analyzed through phosphotyrosine immunoblot. The results are illustrated in FIG. 10.

As illustrated in FIG. 10, it was found that the Lrig-1 protein-specific monoclonal antibodies (A7, C8, E7, and G3) according to the present invention increase phosphorylation of Stat3 to the same level as Th17 cells. On the other hand, it was found that the Lrig-1 protein-specific monoclonal antibodies (A8, B8, D9, and H6) according to the present invention continue to maintain and decrease phosphorylation of Stat3 at the same level as iTreg cells.

### [Example 71 Cancer therapeutic effects of A8, B8, D9, and H6 antibodies

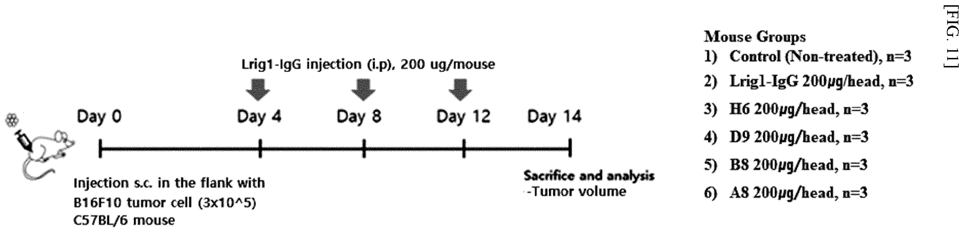

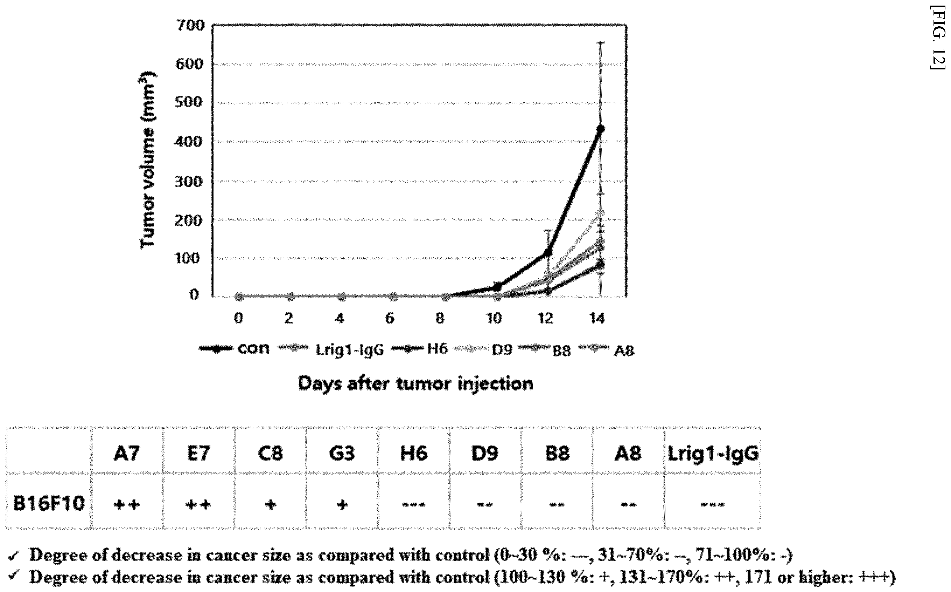

In order to identify therapeutic effects, on solid cancer, of the monoclonal antibodies (A8, B8, D9, and H6) according to the present invention, which had been produced in Production Examples 5 to 8, as illustrated in FIG. 11, B16F10 melanoma cells were subcutaneously injected into the dorsal area of mice in an amount of 3 × 10⁵ cells, and then the antibodies of Production Examples 5 to 8 were intraperitoneally injected into the mice in an amount of 200 ug on days 4, 8, and 12. After transplantation of the melanoma cells, changes in tumor volume over time were measured and the results are illustrated in FIG. 12.

As illustrated in FIG. 12, it was found that remarkably decreased melanoma tumor sizes are observed in a case of being treated with the Lrig-1 protein-specific monoclonal antibodies (A8, B8, D9, and H6) according to the present invention, as compared with a negative control having not received antibody treatment.

From these results, it can be seen that the Lrig-1 protein-specific monoclonal antibodies according to the present invention suppress growth of various solid cancer cells, thereby effectively preventing, ameliorating, or treating such cancers.

### [Example 81 Determination of epitopes in Lrig-1 protein according to present invention

In order to discover epitopes that bind to the H6 antibody of Production Example 8, in which the epitopes are present in respective LRR domains and Ig-like domains in the Lrig-1 protein present on the surface of regulatory T cells, the following experiment was performed.

### 1. Experimental preparation

### (1) Microarray

The Lrig-1 protein was elongated by GSGSGSGlinkers at the C- and N-termini thereof to avoid truncation of peptides. The elongated antigenic sequence was translated into 15 amino acids with a peptide-peptide overlap of 14 amino acids. The resulting Lrig-1 peptide microarrays contained 1,091 different peptides printed in duplicates and framed by additional HA (YPYDVPDYAG, 102 spots) control peptides.

### (2) Experimental material

Sample: H6 monoclonal antibody of Production Example 8
Washing buffer: PBS, pH 7.4 with 0.05% Tween 20 (3 times, 10 seconds after each incubation)
Blocking buffer: Rockland blocking buffer MB-070 (30 minutes before first assay)
Incubation buffer: wash buffer with 10% blocking buffer
Assay conditions: antibody concentration in incubation buffer being adjusted to 1 µg/ml, 10 µg/ml, and 100 µg/ml; and incubation being performed at 4°C for 16 hours, and stirring being performed at 140 rpm
Secondary antibody: goat anti-human IgG (H+L) DyLight680 (0.2 µg/ml); staining being performed in incubation buffer at room temperature for 45 minutes
Control antibody: mouse monoclonal anti-HA (12CA5) DyLight800 (0.5 µg/ml); staining being performed in incubation buffer at room temperature for 45 minutes
Scanner: LI-COR Odyssey Imaging System; scanning offset 0.65 mm, resolution 21 µm, scanning intensity of 7/7 (red = 700 nm/green = 800 nm)

### 2. Experimental method

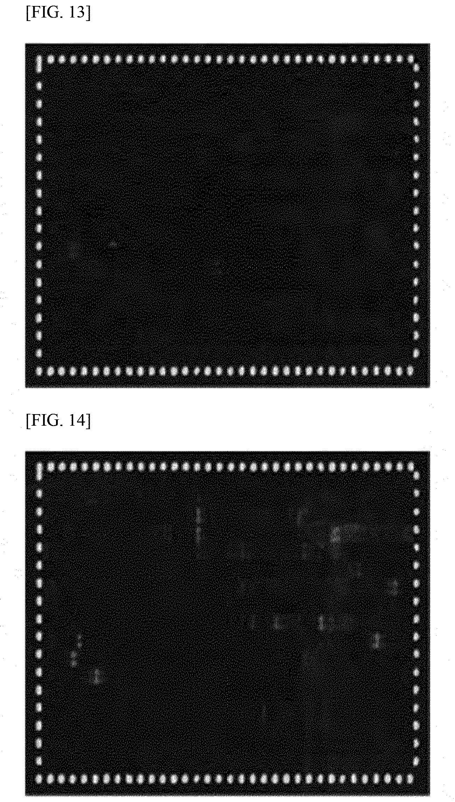

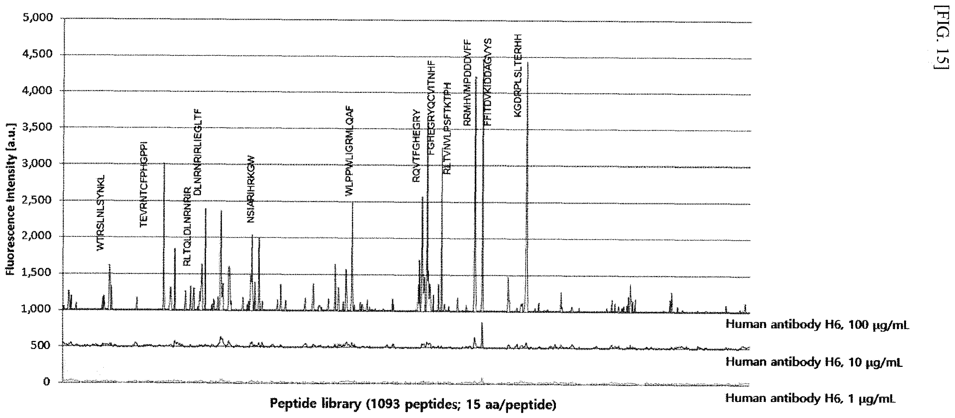

Pre-staining of a Lrig-1 peptide microarray copy using secondary and control antibodies in incubation buffer was performed to identify whether they can interact with antigen-derived peptides that can interfere with the main assays. Subsequent incubation of other Lrig-1 peptide microarray copies with the H6 monoclonal antibody of Production Example 8 (1 µg/ml, 10 µg/ml, and 100 µg/ml) in incubation buffer was followed by staining with secondary and control antibodies. Read-out was performed at a scanning intensity of 7/7 (red/green) using the LI-COR Odyssey Imaging System. Quantification of spot intensities and peptide annotation were based on the 16-bit gray scale tiff files. Microarray image analysis was done with PepSlide® Analyzer. The results are illustrated in FIGS. 13 to 15, and the analyzed epitope sequences are shown in Table 8 below.

**[Table 8]**

| SEQ ID NO | Sequence information |
|---|---|
| SEQ ID NO: 18 | WTRSLNLSYNKL |
| SEQ ID NO: 19 | TEVRNTCFPHGPPI |
| SEQ ID NO: 20 | RLTQLDLNRNRIR |
| SEQ ID NO: 21 | DLNRNRIRLIEGLTF |
| SEQ ID NO: 22 | NSIARIHRKGW |
| SEQ ID NO: 23 | WLPPWLIGRMLQAF |
| SEQ ID NO: 24 | RQVTFGHEGRY |
| SEQ ID NO: 25 | FGHEGRYQCVITNHF |
| SEQ ID NO: 26 | RLTVNVLPF TKTPH |
| SEQ ID NO: 27 | RRMHVMPDDDVFF |
| SEQ ID NO: 28 | FFITDVKIDDAGVYS |
| SEQ ID NO: 29 | KGDRPLSLTERHH |

### 3. Experimental results

As illustrated in FIGS. 13 to 15, it was found that the monoclonal antibody (H6), which specifically binds to Lrig-1 protein present on regulatory T cells and thus effectively treats cancer, specifically binds to an epitope represented by any one amino acid sequence of SEQ ID NOs: 18 to 29 in the Lrig-1 protein and thus exerts such a function. Furthermore, it was found that the epitopes represented by SEQ ID NOs: 18 and 20 have a leucine-rich repeat in common, and specifically, these epitopes contain a consensus sequence, which may be represented by the amino acid sequence of Formula 1 of the present invention, at the fifth amino acid position from the N-terminus.

Although the present invention has been described in detail above, the scope of the present invention is not limited thereto. It will be obvious to those skilled in the art that various modifications and changes can be made without departing from the technical spirit of the present invention described in the claims.

### Industrial availability

The present invention relates to an epitope of leucine-rich and immunoglobulin-like domains 1 (Lrig-1) protein, which is an antigen present on the surface of regulatory T cells, and an antibody or antigen-binding fragment specifically binding thereto.

## Claims

1. An antibody or antigen-binding fragment which specifically binds to an epitope including a polypeptide that consists of an amino acid sequence represented by Formula 1,
[Formula 1] Lx¹Lx²x³N
(in Formula 1, x¹ to x³ are each independently a neutral amino acid, an acidic amino acid, a basic amino acid, or an aromatic amino acid).

2. The antibody or antigen-binding fragment according to claim 1,
wherein x¹ to x³ are each independently an amino acid selected from the group consisting of asparagine (N), aspartic acid (D), serine (S), tyrosine (Y), arginine (R), phenylalanine (F), lysine (K), histidine (H), leucine (L), valine (V), threonine (T), alanine (A), glutamine (Q), glutamic acid (E), and glycine (G).

3. The antibody or antigen-binding fragment according to claim 1,
wherein x¹ is an amino acid selected from the group consisting of asparagine (N), phenylalanine (F), aspartic acid (D), lysine (K), histidine (H), valine (V), arginine (R), and threonine (T),
x² is an amino acid selected from the group consisting of serine (S), glutamine (Q), alanine (A), asparagine (N), glutamic acid (E), aspartic acid (D), phenylalanine (F), and glycine (G), and
x³ is an amino acid selected from the group consisting of tyrosine (Y), histidine (H), glycine (G), arginine (R), asparagine (N), leucine (L), lysine (K), and phenylalanine (F).

4. The antibody or antigen-binding fragment according to claim 1,
wherein the epitope consists of 10- to 20-mer.

5. The antibody or antigen-binding fragment according to claim 1,
wherein the polypeptide that consists of the amino acid sequence represented by Formula 1 is located at positions 3 to 6 from the N-terminus of the epitope.

6. The antibody or antigen-binding fragment according to claim 1,
wherein the polypeptide is represented by any one amino acid sequence of SEQ ID NOs: 4 to 17.

7. An antibody or antigen-binding fragment which specifically binds to an epitope of Lrig-1 protein represented by any one amino acid sequence of SEQ ID NOs: 18 to 29.

8. An epitope of leucine-rich and immunoglobulin-like domains 1 (Lrig-1) protein, comprising:
a polypeptide consisting of an amino acid sequence represented by Formula 1,
[Formula 1] Lx¹Lx²x³N
(in Formula 1, x¹ to x³ are each independently a neutral amino acid, an acidic amino acid, a basic amino acid, or an aromatic amino acid).

9. An epitope of leucine-rich and immunoglobulin-like domains 1 (Lrig-1) protein, comprising:
a polypeptide consisting of any one amino acid sequence of SEQ ID NOs: 18 to 29.

10. A nucleic acid molecule which encodes the epitope according to claim 9.

11. An expression vector into which the nucleic acid molecule according to claim 10 is inserted.

12. A host cell line, transfected with the expression vector according to claim 11.

13. A pharmaceutical composition for preventing or treating cancer, comprising as an active ingredient:
the antibody or antigen-binding fragment according to any one of claims 1 to 6.

14. The pharmaceutical composition according to claim 13,
wherein the cancer is gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer.

15. A pharmaceutical composition for preventing or treating cancer, comprising as an active ingredient:
the antibody or antigen-binding fragment according to claim 7.

16. The pharmaceutical composition according to claim 15,
wherein the cancer is gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer.

17. An antibody-drug conjugate, comprising:
the antibody or antigen-binding fragment according to any one of claims 1 to 6; and
a drug.

18. An antibody-drug conjugate, comprising:
the antibody or antigen-binding fragment according to claim 7; and
a drug.

19. A method for preventing or treating cancer, comprising:
a step of administering, to an individual, the antibody or antigen-binding fragment according to any one of claims 1 to 6.

20. A method for preventing or treating cancer, comprising:
a step of administering, to an individual, the antibody or antigen-binding fragment according to claim 7.


<110>    Good T Cells, Inc.

<120>    Epitope of regulatory T cell surface antigen and an antibody
         specifically binding to the epitope thereof

<130>    PDPB187156k01

<150>    KR 10-2018-0053052
<151>    2018-05-09

<160>    45

<170>    KoPatentIn 3.0

<210>    1
<211>    1093
<212>    PRT
<213>    Homo sapiens

<400>    1


<210>    2
<211>    3282
<212>    DNA
<213>    Homo sapiens

<400>    2
   atggcgcggc cggtccgggg agggctcggg gccccgcgcc gctcgccttg ccttctcctt      60
   ctctggctgc ttttgcttcg gctggagccg gtgaccgccg cggccggccc gcgggcgccc     120
   tgcgcggccg cctgcacttg cgctggggac tcgctggact gcggtgggcg cgggctggct     180
   gcgttgcccg gggacctgcc ctcctggacg cggagcctaa acctgagtta caacaaactc     240
   tctgagattg accctgctgg ttttgaggac ttgccgaacc tacaggaagt gtacctcaat     300
   aataatgagt tgacagcggt accatccctg ggcgctgctt catcacatgt cgtctctctc     360
   tttctgcagc acaacaagat tcgcagcgtg gaggggagcc agctgaaggc ctacctttcc     420
   ttagaagtgt tagatctgag tttgaacaac atcacggaag tgcggaacac ctgctttcca     480
   cacggaccgc ctataaagga gctcaacctg gcaggcaatc ggattggcac cctggagttg     540
   ggagcatttg atggtctgtc acggtcgctg ctaactcttc gcctgagcaa aaacaggatc 600
   acccagcttc ctgtaagagc attcaagcta cccaggctga cacaactgga cctcaatcgg 660
   aacaggattc ggctgataga gggcctcacc ttccaggggc tcaacagctt ggaggtgctg 720
   aagcttcagc gaaacaacat cagcaaactg acagatgggg ccttctgggg actgtccaag 780
   atgcatgtgc tgcacctgga gtacaacagc ctggtagaag tgaacagcgg ctcgctctac 840
   ggcctcacgg ccctgcatca gctccacctc agcaacaatt ccatcgctcg cattcaccgc 900
   aagggctgga gcttctgcca gaagctgcat gagttggtcc tgtccttcaa caacctgaca 960
   cggctggacg aggagagcct ggccgagctg agcagcctga gtgtcctgcg tctcagccac 1020
   aattccatca gccacattgc ggagggtgcc ttcaagggac tcaggagcct gcgagtcttg 1080
   gatctggacc ataacgagat ttcgggcaca atagaggaca cgagcggcgc cttctcaggg 1140
   ctcgacagcc tcagcaagct gactctgttt ggaaacaaga tcaagtctgt ggctaagaga 1200
   gcattctcgg ggctggaagg cctggagcac ctgaaccttg gagggaatgc gatcagatct 1260
   gtccagtttg atgcctttgt gaagatgaag aatcttaaag agctccatat cagcagcgac 1320
   agcttcctgt gtgactgcca gctgaagtgg ctgcccccgt ggctaattgg caggatgctg 1380
   caggcctttg tgacagccac ctgtgcccac ccagaatcac tgaagggtca gagcattttc 1440
   tctgtgccac cagagagttt cgtgtgcgat gacttcctga agccacagat catcacccag 1500
   ccagaaacca ccatggctat ggtgggcaag gacatccggt ttacatgctc agcagccagc 1560
   agcagcagct cccccatgac ctttgcctgg aagaaagaca atgaagtcct gaccaatgca 1620
   gacatggaga actttgtcca cgtccacgcg caggacgggg aagtgatgga gtacaccacc 1680
   atcctgcacc tccgtcaggt cactttcggg cacgaggccc gctaccaatg tgtcatcacc 1740
   aaccactttg gctccaccta ttcacataag gccaggctca ccgtgaatgt gttgccatca 1800
   ttcaccaaaa cgccccacga cataaccatc cggaccacca ccatggcccg cctcgaatgt 1860
   gctgccacag gtcacccaaa ccctcagatt gcctggcaga aggatggagg cacggatttc 1920
   cccgctgccc gtgagcgacg catgcatgtc atgccggatg acgacgtgtt tttcatcact 1980
   gatgtgaaaa tagatgacgc aggggtttac agctgtactg ctcagaactc agccggttct 2040
   atttcagcta atgccaccct gactgtccta gagaccccat ccttggtggt ccccttggaa 2100
   gaccgtgtgg tatctgtggg agaaacagtg gccctccaat gcaaagccac ggggaaccct 2160
   ccgccccgca tcacctggtt caagggggac cgcccgctga gcctcactga gcggcaccac 2220
   ttgacccctg acaaccagct cctggtggtt cagaacgtgg tggcagagga tgcgggccga 2280
   tatacctgtg agatgtccaa caccctgggc acggagcgag ctcacagcca gctgagcgtc 2340
   ctgcccgcag caggctgcag gaaggatggg accacggtag gcatcttcac cattgctgtc 2400
   gtgagcagca tcgtcctgac gtcactggtc tgggtgtgca tcatctacca gaccaggaag 2460
   aagagtgaag agtacagtgt caccaacaca gatgaaaccg tcgtgccacc agatgttcca 2520
   agctacctct cttctcaggg gaccctttct gaccgacaag aaaccgtggt caggaccgag 2580
   ggtggccctc aggccaatgg gcacattgag agcaatggtg tgtgtccaag agatgcaagc 2640
   cactttccag agcccgacac tcacagcgtt gcctgcaggc agccaaagct ctgtgctggg 2700
   tctgcgtatc acaaagagcc gtggaaagcg atggagaaag ctgaagggac acctgggcca 2760
   cataagatgg aacacggtgg ccgggtcgta tgcagtgact gcaacaccga agtggactgt 2820
   tactccaggg gacaagcctt ccacccccag cctgtgtcca gagacagcgc acagccaagt 2880
   gcgccaaatg gcccggagcc gggtgggagt gaccaagagc attctccaca tcaccagtgc 2940
   agcaggactg ccgctgggtc ctgccccgag tgccaagggt cgctctaccc cagtaaccac 3000
   gatagaatgc tgacggctgt gaagaaaaag ccaatggcat ctctagatgg gaaaggggat 3060
   tcttcctgga ctttagcaag gttgtatcac ccggactcca cagagctaca gcctgcatct 3120
   tcattaactt caggcagtcc agagcgcgcg gaagcccagt acttgcttgt ttccaatggc 3180
   cacctcccca aagcatgtga cgccagtccc gagtccacgc cactgacagg acagctcccc 3240
   gggaaacaga gggtgccact gctgttggca ccaaaaagct ag 3282

<210> 3
<211> 1091
<212> PRT
<213> Mus musculus

<400> 3
Met Ala Arg Pro Gly Pro Gly Val Leu Gly Ala Pro Arg Leu Ala Pro
1               5                   10                  15

Arg Leu Leu Leu Trp Leu Leu Leu Leu Leu Leu Gln Trp Pro Glu Ser
            20                  25                  30

Ala Gly Ala Gln Ala Gly Pro Arg Ala Pro Cys Ala Ala Ala Cys Thr
        35                  40                  45

Cys Ala Gly Asp Ser Leu Asp Cys Ser Gly Arg Gly Leu Ala Thr Leu
    50                  55                  60

Pro Arg Asp Leu Pro Ser Trp Thr Arg Ser Leu Asn Leu Ser Tyr Asn
65                  70                  75                  80

Arg Leu Ser Glu Ile Asp Ser Ala Ala Phe Glu Asp Leu Thr Asn Leu
                85                  90                  95

Gln Glu Val Tyr Leu Asn Ser Asn Glu Leu Thr Ala Ile Pro Ser Leu
            100                 105                 110

Gly Thr Ala Ser Ile Gly Val Val Ser Leu Phe Leu Gln His Asn Lys
        115                 120                 125

Ile Leu Ser Val Asp Gly Ser Gln Leu Lys Ser Tyr Leu Ser Leu Glu
    130                 135                 140

Val Leu Asp Leu Ser Ser Asn Asn Ile Thr Glu Ile Arg Ser Ser Cys
145                 150                 155                 160

Phe Pro Asn Gly Leu Arg Ile Arg Glu Leu Asn Leu Ala Ser Asn Arg
                165                 170                 175

Ile Ser Ile Leu Glu Ser Gly Ala Phe Asp Gly Leu Ser Arg Ser Leu
            180                 185                 190

Leu Thr Leu Arg Leu Ser Lys Asn Arg Ile Thr Gln Leu Pro Val Lys
        195                 200                 205

Ala Phe Lys Leu Pro Arg Leu Thr Gln Leu Asp Leu Asn Arg Asn Arg
    210                 215                 220

Ile Arg Leu Ile Glu Gly Leu Thr Phe Gln Gly Leu Asp Ser Leu Glu
225                 230                 235                 240

Val Leu Arg Leu Gln Arg Asn Asn Ile Ser Arg Leu Thr Asp Gly Ala
                245                 250                 255

Phe Trp Gly Leu Ser Lys Met His Val Leu His Leu Glu Tyr Asn Ser
            260                 265                 270

Leu Val Glu Val Asn Ser Gly Ser Leu Tyr Gly Leu Thr Ala Leu His
        275                 280                 285

Gln Leu His Leu Ser Asn Asn Ser Ile Ser Arg Ile Gln Arg Asp Gly
    290                 295                 300

Trp Ser Phe Cys Gln Lys Leu His Glu Leu Ile Leu Ser Phe Asn Asn
305                 310                 315                 320

Leu Thr Arg Leu Asp Glu Glu Ser Leu Ala Glu Leu Ser Ser Leu Ser
                325                 330                 335

Ile Leu Arg Leu Ser His Asn Ala Ile Ser His Ile Ala Glu Gly Ala
            340                 345                 350

Phe Lys Gly Leu Lys Ser Leu Arg Val Leu Asp Leu Asp His Asn Glu
        355                 360                 365

Ile Ser Gly Thr Ile Glu Asp Thr Ser Gly Ala Phe Thr Gly Leu Asp
    370                 375                 380

Asn Leu Ser Lys Leu Thr Leu Phe Gly Asn Lys Ile Lys Ser Val Ala
385                 390                 395                 400

Lys Arg Ala Phe Ser Gly Leu Glu Ser Leu Glu His Leu Asn Leu Gly
                405                 410                 415

Glu Asn Ala Ile Arg Ser Val Gln Phe Asp Ala Phe Ala Lys Met Lys
            420                 425                 430

Asn Leu Lys Glu Leu Tyr Ile Ser Ser Glu Ser Phe Leu Cys Asp Cys
        435                 440                 445

Gln Leu Lys Trp Leu Pro Pro Trp Leu Met Gly Arg Met Leu Gln Ala
    450                 455                 460

Phe Val Thr Ala Thr Cys Ala His Pro Glu Ser Leu Lys Gly Gln Ser
465                 470                 475                 480

Ile Phe Ser Val Leu Pro Asp Ser Phe Val Cys Asp Asp Phe Pro Lys
                485                 490                 495

Pro Gln Ile Ile Thr Gln Pro Glu Thr Thr Met Ala Val Val Gly Lys
            500                 505                 510

Asp Ile Arg Phe Thr Cys Ser Ala Ala Ser Ser Ser Ser Ser Pro Met
        515                 520                 525

Thr Phe Ala Trp Lys Lys Asp Asn Glu Val Leu Ala Asn Ala Asp Met
    530                 535                 540

Glu Asn Phe Ala His Val Arg Ala Gln Asp Gly Glu Val Met Glu Tyr
545                 550                 555                 560

Thr Thr Ile Leu His Leu Arg His Val Thr Phe Gly His Glu Gly Arg
                565                 570                 575

Tyr Gln Cys Ile Ile Thr Asn His Phe Gly Ser Thr Tyr Ser His Lys
            580                 585                 590

Ala Arg Leu Thr Val Asn Val Leu Pro Ser Phe Thr Lys Ile Pro His
        595                 600                 605

Asp Ile Ala Ile Arg Thr Gly Thr Thr Ala Arg Leu Glu Cys Ala Ala
    610                 615                 620

Thr Gly His Pro Asn Pro Gln Ile Ala Trp Gln Lys Asp Gly Gly Thr
625                 630                 635                 640

Asp Phe Pro Ala Ala Arg Glu Arg Arg Met His Val Met Pro Asp Asp
                645                 650                 655

Asp Val Phe Phe Ile Thr Asp Val Lys Ile Asp Asp Met Gly Val Tyr
            660                 665                 670

Ser Cys Thr Ala Gln Asn Ser Ala Gly Ser Val Ser Ala Asn Ala Thr
        675                 680                 685

Leu Thr Val Leu Glu Thr Pro Ser Leu Ala Val Pro Leu Glu Asp Arg
    690                 695                 700

Val Val Thr Val Gly Glu Thr Val Ala Phe Gln Cys Lys Ala Thr Gly
705                 710                 715                 720

Ser Pro Thr Pro Arg Ile Thr Trp Leu Lys Gly Gly Arg Pro Leu Ser
                725                 730                 735

Leu Thr Glu Arg His His Phe Thr Pro Gly Asn Gln Leu Leu Val Val
            740                 745                 750

Gln Asn Val Met Ile Asp Asp Ala Gly Arg Tyr Thr Cys Glu Met Ser
        755                 760                 765

Asn Pro Leu Gly Thr Glu Arg Ala His Ser Gln Leu Ser Ile Leu Pro
    770                 775                 780

Thr Pro Gly Cys Arg Lys Asp Gly Thr Thr Val Gly Ile Phe Thr Ile
785                 790                 795                 800

Ala Val Val Cys Ser Ile Val Leu Thr Ser Leu Val Trp Val Cys Ile
                805                 810                 815

Ile Tyr Gln Thr Arg Lys Lys Ser Glu Glu Tyr Ser Val Thr Asn Thr
            820                 825                 830

Asp Glu Thr Ile Val Pro Pro Asp Val Pro Ser Tyr Leu Ser Ser Gln
        835                 840                 845

Gly Thr Leu Ser Asp Arg Gln Glu Thr Val Val Arg Thr Glu Gly Gly
    850                 855                 860

His Gln Ala Asn Gly His Ile Glu Ser Asn Gly Val Cys Leu Arg Asp
865                 870                 875                 880

Pro Ser Leu Phe Pro Glu Val Asp Ile His Ser Thr Thr Cys Arg Gln
                885                 890                 895

Pro Lys Leu Cys Val Gly Tyr Thr Arg Glu Pro Trp Lys Val Thr Glu
            900                 905                 910

Lys Ala Asp Arg Thr Ala Ala Pro His Thr Thr Ala His Ser Gly Ser
        915                 920                 925

Ala Val Cys Ser Asp Cys Ser Thr Asp Thr Ala Tyr His Pro Gln Pro
    930                 935                 940

Val Pro Arg Asp Ser Gly Gln Pro Gly Thr Ala Ser Ser Gln Glu Leu
945                 950                 955                 960

Arg Gln His Asp Arg Glu Tyr Ser Pro His His Pro Tyr Ser Gly Thr
                965                 970                 975

Ala Asp Gly Ser His Thr Leu Ser Gly Gly Ser Leu Tyr Pro Ser Asn
            980                 985                 990

His Asp Arg Ile Leu Pro Ser Leu Lys Asn Lys Ala Ala Ser Ala Asp
        995                 1000                1005

Gly Asn Gly Asp Ser Ser Trp Thr Leu Ala Lys Leu His Glu Ala Asp
    1010                1015                1020

Cys Ile Asp Leu Lys Pro Ser Pro Thr Leu Ala Ser Gly Ser Pro Glu
1025                1030                1035                1040

Leu Met Glu Asp Ala Ile Ser Thr Glu Ala Gln His Leu Leu Val Ser
                1045                1050                1055

Asn Gly His Leu Pro Lys Ala Cys Asp Ser Ser Pro Glu Ser Val Pro
            1060                1065                1070

Leu Lys Gly Gln Ile Thr Gly Lys Arg Arg Gly Pro Leu Leu Leu Ala
        1075                1080                1085

Pro Arg Ser
    1090


<210>   4
<211>   6
<212>   PRT
<213>   Artificial Sequence

<220>
<223>   Lrig-1 epitope

<400>    4
Leu Asn Leu Ser Tyr Asn
  1               5

<210>    5
<211>    6
<212>    PRT
<213>    Artificial Sequence

<220>
<223>    Lrig-1 epitope

<400>    5
Leu Asp Leu Asn Arg Asn
  1               5

<210>    6
<211>    6
<212>    PRT
<213>    Artificial Sequence

<220>
<223>    Lrig-1 epitope

<400>    6
Leu Phe Leu Gln His Asn
  1               5

<210>    7
<211>    6
<212>    PRT
<213>    Artificial Sequence

<220>
<223>    Lrig-1 epitope

<400>    7
Leu Asn Leu Ala Gly Asn
  1               5

<210>    8
<211>    6
<212>    PRT
<213>    Artificial Sequence

<220>
<223>    Lrig-1 epitope

<400>    8
Leu Asp Leu Ser Leu Asn
  1               5

<210>    9

<211>    6
<212>    PRT
<213>    Artificial Sequence

<220>
<223>    Lrig-1 epitope


<400>    9
Leu Arg Leu Ser Lys Asn
1               5


<210>    10
<211>    6
<212>    PRT
<213>    Artificial Sequence

<220>
<223>    Lrig-1 epitope


<400>    10
Leu Lys Leu Gln Arg Asn
1               5


<210>    11
<211>    6
<212>    PRT
<213>    Artificial Sequence

<220>
<223>    Lrig-1 epitope


<400>    11
Leu His Leu Glu Tyr Asn
1               5


<210>    12
<211>    6
<212>    PRT
<213>    Artificial Sequence

<220>
<223>    Lrig-1 epitope


<400>    12
Leu His Leu Ser Asn Asn
1               5


<210>    13
<211>    6
<212>    PRT
<213>    Artificial Sequence

<220>
<223>    Lrig-1 epitope

<400>    13
Leu Val Leu Ser Phe Asn
1               5

<210>    14
<211>    6
<212>    PRT
<213>    Artificial Sequence

<220>
<223>    Lrig-1 epitope

<400>    14
Leu Arg Leu Ser His Asn
1               5

<210>    15
<211>    6
<212>    PRT
<213>    Artificial Sequence

<220>
<223>    Lrig-1 epitope

<400>    15
Leu Asp Leu Asp His Asn
1               5

<210>    16
<211>    6
<212>    PRT
<213>    Artificial Sequence

<220>
<223>    Lrig-1 epitope

<400>    16
Leu Thr Leu Phe Gly Asn
1               5

<210>    17
<211>    6
<212>    PRT
<213>    Artificial Sequence

<220>
<223>    Lrig-1 epitope

<400>    17
Leu Asn Leu Gly Gly Asn
1               5

<210>    18

<211>    12
<212>    PRT
<213>    Artificial Sequence

<220>
<223>    Lrig-1 epitope


<400>    18
Trp Thr Arg Ser Leu Asn Leu Ser Tyr Asn Lys Leu
1               5                   10


<210>    19
<211>    14
<212>    PRT
<213>    Artificial Sequence

<220>
<223>    Lrig-1 epitope


<400>    19
Thr Glu Val Arg Asn Thr Cys Phe Pro His Gly Pro Pro Ile
1               5                   10


<210>    20
<211>    13
<212>    PRT
<213>    Artificial Sequence

<220>
<223>    Lrig-1 epitope


<400>    20
Arg Leu Thr Gln Leu Asp Leu Asn Arg Asn Arg Ile Arg
1               5                   10


<210>    21
<211>    15
<212>    PRT
<213>    Artificial Sequence

<220>
<223>    Lrig-1 epitope


<400>    21
Asp Leu Asn Arg Asn Arg Ile Arg Leu Ile Glu Gly Leu Thr Phe
1               5                   10                  15


<210>    22
<211>    11
<212>    PRT
<213>    Artificial Sequence

<220>
<223>    Lrig-1 epitope

<400>    22
Asn Ser Ile Ala Arg Ile His Arg Lys Gly Trp
1               5                   10

<210>    23
<211>    14
<212>    PRT
<213>    Artificial Sequence

<220>
<223>    Lrig-1 epitope

<400>    23
Trp Leu Pro Pro Trp Leu Ile Gly Arg Met Leu Gln Ala Phe
1               5                   10

<210>    24
<211>    11
<212>    PRT
<213>    Artificial Sequence

<220>
<223>    Lrig-1 epitope

<400>    24
Arg Gln Val Thr Phe Gly His Glu Gly Arg Tyr
1               5                   10

<210>    25
<211>    15
<212>    PRT
<213>    Artificial Sequence

<220>
<223>    Lrig-1 epitope

<400>    25
Phe Gly His Glu Gly Arg Tyr Gln Cys Val Ile Thr Asn His Phe
1               5                   10                  15

<210>    26
<211>    15
<212>    PRT
<213>    Artificial Sequence

<220>
<223>    Lrig-1 epitope

<400>    26
Arg Leu Thr Val Asn Val Leu Pro Ser Phe Thr Lys Thr Pro His
1               5                   10                  15

<210>    27

<211>    13
<212>    PRT
<213>    Artificial Sequence

<220>
<223>    Lrig-1 epitope


<400>    27
Arg Arg Met His Val Met Pro Asp Asp Asp Val Phe Phe
1               5                   10


<210>    28
<211>    15
<212>    PRT
<213>    Artificial Sequence

<220>
<223>    Lrig-1 epitope


<400>    28
Phe Phe Ile Thr Asp Val Lys Ile Asp Asp Ala Gly Val Tyr Ser
1               5                   10                  15


<210>    29
<211>    13
<212>    PRT
<213>    Artificial Sequence

<220>
<223>    Lrig-1 epitope


<400>    29
Lys Gly Asp Arg Pro Leu Ser Leu Thr Glu Arg His His
1               5                   10


<210>    30
<211>    449
<212>    PRT
<213>    Artificial Sequence

<220>
<223>    A7 heavy chain


<400>    30
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Asp Met Ser Trp Val Arg Gln Ala Pro Gly Lys Gly Leu Glu Trp Val
        35                  40                  45

Ser Leu Ile Tyr Pro Asp Ser Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ala Gly Leu Ser Trp Ala Gly Ala Phe Asp Tyr Trp Gly
            100                 105                 110

Gln Gly Thr Leu Val Thr Val Ser Ser Thr Thr Ala Pro Ser Val Tyr
        115                 120                 125

Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Thr Ser Ser
            180                 185                 190

Thr Trp Pro Ser Gln Ser Ile Thr Cys Asn Val Ala His Pro Ala Ser
        195                 200                 205

Ser Thr Lys Val Asp Lys Lys Ile Glu Pro Arg Gly Pro Thr Ile Lys
    210                 215                 220

Pro Cys Pro Pro Cys Lys Cys Pro Ala Pro Asn Leu Leu Gly Gly Pro
225                 230                 235                 240

Ser Val Phe Ile Phe Pro Pro Lys Ile Lys Asp Val Leu Met Ile Ser
                245                 250                 255

Leu Ser Pro Ile Val Thr Cys Val Val Val Asp Val Ser Glu Asp Asp
            260                 265                 270

Pro Asp Val Gln Ile Ser Trp Phe Val Asn Asn Val Glu Val His Thr
        275                 280                 285

Ala Gln Thr Gln Thr His Arg Glu Asp Tyr Asn Ser Thr Leu Arg Val
    290                 295                 300

Val Ser Ala Leu Pro Ile Gln His Gln Asp Trp Met Ser Gly Lys Glu
305                 310                 315                 320

Phe Lys Cys Lys Val Asn Asn Lys Asp Leu Pro Ala Pro Ile Glu Arg
                325                 330                 335

Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365

Cys Met Val Thr Asp Phe Met Pro Glu Asp Ile Tyr Val Glu Trp Thr
    370                 375                 380

Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415

Lys Asn Trp Val Glu Arg Asn Ser Tyr Ser Cys Ser Val Val His Glu
            420                 425                 430

Gly Leu His Asn His His Thr Thr Lys Ser Phe Ser Arg Thr Pro Gly
        435                 440                 445

Lys

<210>    31
<211>    217
<212>    PRT
<213>    Artificial Sequence

<220>
<223>    A7 light chain

<400>    31
Gln Ser Val Leu Thr Gln Pro Pro Ser Ala Ser Gly Thr Pro Gly Gln
  1               5                  10                  15

Arg Val Thr Ile Ser Cys Ser Gly Ser Ser Ser Asn Ile Gly Ser Asn
             20                  25                  30

Tyr Val Thr Trp Tyr Gln Gln Leu Pro Gly Thr Ala Pro Lys Leu Leu
         35                  40                  45

Ile Tyr Ser Asp Ser His Arg Pro Ser Gly Val Pro Asp Arg Phe Ser
     50                  55                  60

Gly Ser Lys Ser Gly Thr Ser Ala Ser Leu Ala Ile Ser Gly Leu Gln
 65                  70                  75                  80

Ser Glu Asp Glu Ala Asp Tyr Tyr Cys Gly Ser Trp Asp Tyr Ser Leu
                 85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys

210                 215

<210>   32
<211>   449
<212>   PRT
<213>   Artificial Sequence

<220>
<223>   C8 heavy chain

<400>   32
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Lys Gly Leu Glu Trp Val
        35                  40                  45

Ser Gly Ile Ser Pro Gly Asp Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Leu Tyr Ser Asn Pro Asn Glu Pro Phe Asp Tyr Trp Gly
            100                 105                 110

Gln Gly Thr Leu Val Thr Val Ser Ser Thr Thr Ala Pro Ser Val Tyr
        115                 120                 125

Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Thr Ser Ser
            180                 185                 190

Thr Trp Pro Ser Gln Ser Ile Thr Cys Asn Val Ala His Pro Ala Ser
        195                 200                 205

Ser Thr Lys Val Asp Lys Lys Ile Glu Pro Arg Gly Pro Thr Ile Lys
    210                 215                 220

Pro Cys Pro Pro Cys Lys Cys Pro Ala Pro Asn Leu Leu Gly Gly Pro
225                 230                 235                 240

Ser Val Phe Ile Phe Pro Pro Lys Ile Lys Asp Val Leu Met Ile Ser
                245                 250                 255

Leu Ser Pro Ile Val Thr Cys Val Val Val Asp Val Ser Glu Asp Asp
            260                 265                 270

Pro Asp Val Gln Ile Ser Trp Phe Val Asn Asn Val Glu Val His Thr
        275                 280                 285

Ala Gln Thr Gln Thr His Arg Glu Asp Tyr Asn Ser Thr Leu Arg Val
    290                 295                 300

Val Ser Ala Leu Pro Ile Gln His Gln Asp Trp Met Ser Gly Lys Glu
305                 310                 315                 320

Phe Lys Cys Lys Val Asn Asn Lys Asp Leu Pro Ala Pro Ile Glu Arg
                325                 330                 335

Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365

Cys Met Val Thr Asp Phe Met Pro Glu Asp Ile Tyr Val Glu Trp Thr
    370                 375                 380

Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415

Lys Asn Trp Val Glu Arg Asn Ser Tyr Ser Cys Ser Val Val His Glu
            420                 425                 430

Gly Leu His Asn His His Thr Thr Lys Ser Phe Ser Arg Thr Pro Gly
        435                 440                 445

Lys

<210>   33
<211>   217
<212>   PRT
<213>   Artificial Sequence

<220>
<223>   C8 light chain

<400>   33
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Asn Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>   34
<211>   455
<212>   PRT
<213>   Artificial Sequence

<220>
<223>   E7 heavy chain


<400>   34
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ser Gly Ile Ser Pro Asp Gly Ser Asn Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Gly Leu Arg Cys Arg Tyr Glu Ala Cys Ser Tyr Ala Tyr
            100                 105                 110

Gly Met Asp Val Trp Gly Gln Gly Thr Leu Val Thr Val Ser Ser Thr
        115                 120                 125

Thr Ala Pro Ser Val Tyr Pro Leu Ala Pro Val Cys Gly Asp Thr Thr

130                 135                 140

Gly Ser Ser Val Thr Leu Gly Cys Leu Val Lys Gly Tyr Phe Pro Glu
145                 150                 155                 160

Pro Val Thr Leu Thr Trp Asn Ser Gly Ser Leu Ser Ser Gly Val His
                165                 170                 175

Thr Phe Pro Ala Val Leu Gln Ser Asp Leu Tyr Thr Leu Ser Ser Ser
            180                 185                 190

Val Thr Val Thr Ser Ser Thr Trp Pro Ser Gln Ser Ile Thr Cys Asn
        195                 200                 205

Val Ala His Pro Ala Ser Ser Thr Lys Val Asp Lys Lys Ile Glu Pro
    210                 215                 220

Arg Gly Pro Thr Ile Lys Pro Cys Pro Pro Cys Lys Cys Pro Ala Pro
225                 230                 235                 240

Asn Leu Leu Gly Gly Pro Ser Val Phe Ile Phe Pro Pro Lys Ile Lys
                245                 250                 255

Asp Val Leu Met Ile Ser Leu Ser Pro Ile Val Thr Cys Val Val Val
            260                 265                 270

Asp Val Ser Glu Asp Asp Pro Asp Val Gln Ile Ser Trp Phe Val Asn
        275                 280                 285

Asn Val Glu Val His Thr Ala Gln Thr Gln Thr His Arg Glu Asp Tyr
    290                 295                 300

Asn Ser Thr Leu Arg Val Val Ser Ala Leu Pro Ile Gln His Gln Asp
305                 310                 315                 320

Trp Met Ser Gly Lys Glu Phe Lys Cys Lys Val Asn Asn Lys Asp Leu
                325                 330                 335

Pro Ala Pro Ile Glu Arg Thr Ile Ser Lys Pro Lys Gly Ser Val Arg
            340                 345                 350

Ala Pro Gln Val Tyr Val Leu Pro Pro Pro Glu Glu Glu Met Thr Lys
        355                 360                 365

Lys Gln Val Thr Leu Thr Cys Met Val Thr Asp Phe Met Pro Glu Asp
    370                 375                 380

Ile Tyr Val Glu Trp Thr Asn Asn Gly Lys Thr Glu Leu Asn Tyr Lys
385                 390                 395                 400

Asn Thr Glu Pro Val Leu Asp Ser Asp Gly Ser Tyr Phe Met Tyr Ser
                405                 410                 415

Lys Leu Arg Val Glu Lys Lys Asn Trp Val Glu Arg Asn Ser Tyr Ser
            420                 425                 430

Cys Ser Val Val His Glu Gly Leu His Asn His His Thr Thr Lys Ser
        435                 440                 445

Phe Ser Arg Thr Pro Gly Lys
    450                 455

<210>    35
<211>    217
<212>    PRT
<213>    Artificial Sequence

<220>
<223>    E7 light chain

<400>    35
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Thr Trp Asp Ser Ser Leu
                85                  90                  95

Asn Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    36
<211>    449
<212>    PRT
<213>    Artificial Sequence

<220>
<223>    G3 heavy chain


<400>    36

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Asp Met Ser Trp Val Arg Gln Ala Pro Gly Lys Gly Leu Glu Trp Val
        35                  40                  45

Ser Ser Ile Ser Pro Ser Ser Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Leu Asp Ala Phe Trp Arg Pro Ser Phe Asp Tyr Trp Gly
            100                 105                 110

Gln Gly Thr Leu Val Thr Val Ser Ser Thr Thr Ala Pro Ser Val Tyr
        115                 120                 125

Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Thr Ser Ser
            180                 185                 190

Thr Trp Pro Ser Gln Ser Ile Thr Cys Asn Val Ala His Pro Ala Ser
        195                 200                 205

Ser Thr Lys Val Asp Lys Lys Ile Glu Pro Arg Gly Pro Thr Ile Lys
    210                 215                 220

Pro Cys Pro Pro Cys Lys Cys Pro Ala Pro Asn Leu Leu Gly Gly Pro
225                 230                 235                 240

Ser Val Phe Ile Phe Pro Pro Lys Ile Lys Asp Val Leu Met Ile Ser
                245                 250                 255

Leu Ser Pro Ile Val Thr Cys Val Val Val Asp Val Ser Glu Asp Asp
            260                 265                 270

Pro Asp Val Gln Ile Ser Trp Phe Val Asn Asn Val Glu Val His Thr
        275                 280                 285

Ala Gln Thr Gln Thr His Arg Glu Asp Tyr Asn Ser Thr Leu Arg Val
    290                 295                 300

Val Ser Ala Leu Pro Ile Gln His Gln Asp Trp Met Ser Gly Lys Glu
305                 310                 315                 320

Phe Lys Cys Lys Val Asn Asn Lys Asp Leu Pro Ala Pro Ile Glu Arg
                325                 330                 335

Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365

Cys Met Val Thr Asp Phe Met Pro Glu Asp Ile Tyr Val Glu Trp Thr
    370                 375                 380

Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415

Lys Asn Trp Val Glu Arg Asn Ser Tyr Ser Cys Ser Val Val His Glu
            420                 425                 430

Gly Leu His Asn His His Thr Thr Lys Ser Phe Ser Arg Thr Pro Gly
        435                 440                 445

Lys

<210>    37
<211>    217
<212>    PRT
<213>    Artificial Sequence

<220>
<223>    G3 light chain

<400>    37
Gln Ser Val Leu Thr Gln Pro Pro Ser Ala Ser Gly Thr Pro Gly Gln
 1               5                  10                  15

Arg Val Thr Ile Ser Cys Thr Gly Ser Ser Ser Asn Ile Gly Asn Asn
            20                  25                  30

Asn Val Asn Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Asp Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn

145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    38
<211>    455
<212>    PRT
<213>    Artificial Sequence

<220>
<223>    A8 heavy chain


<400>    38
Glu Val Gln Leu Leu Glu Ser Gly Gly Gly Leu Val Gln Pro Gly Gly
 1               5                  10                  15

Ser Leu Arg Leu Ser Cys Ala Ala Ser Gly Phe Thr Phe Ser Asp Tyr
            20                  25                  30

Asp Met Ser Trp Val Arg Gln Val Pro Gly Lys Gly Leu Glu Trp Val
        35                  40                  45

Ser Trp Ile Ser His Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Leu Gly Leu Cys Lys Thr Gly Leu Cys Tyr Tyr Tyr Asp
            100                 105                 110

Ala Met Asp Val Trp Gly Gln Gly Thr Leu Val Thr Val Ser Ser Thr
        115                 120                 125

Thr Ala Pro Ser Val Tyr Pro Leu Ala Pro Val Cys Gly Asp Thr Thr
    130                 135                 140

Gly Ser Ser Val Thr Leu Gly Cys Leu Val Lys Gly Tyr Phe Pro Glu
145                 150                 155                 160

Pro Val Thr Leu Thr Trp Asn Ser Gly Ser Leu Ser Ser Gly Val His
                165                 170                 175

Thr Phe Pro Ala Val Leu Gln Ser Asp Leu Tyr Thr Leu Ser Ser Ser
            180                 185                 190

Val Thr Val Thr Ser Ser Thr Trp Pro Ser Gln Ser Ile Thr Cys Asn
        195                 200                 205

Val Ala His Pro Ala Ser Ser Thr Lys Val Asp Lys Lys Ile Glu Pro
    210                 215                 220

Arg Gly Pro Thr Ile Lys Pro Cys Pro Pro Cys Lys Cys Pro Ala Pro
225                 230                 235                 240

Asn Leu Leu Gly Gly Pro Ser Val Phe Ile Phe Pro Pro Lys Ile Lys
                245                 250                 255

Asp Val Leu Met Ile Ser Leu Ser Pro Ile Val Thr Cys Val Val Val
            260                 265                 270

Asp Val Ser Glu Asp Asp Pro Asp Val Gln Ile Ser Trp Phe Val Asn
        275                 280                 285

Asn Val Glu Val His Thr Ala Gln Thr Gln Thr His Arg Glu Asp Tyr
    290                 295                 300

Asn Ser Thr Leu Arg Val Val Ser Ala Leu Pro Ile Gln His Gln Asp
305                 310                 315                 320

Trp Met Ser Gly Lys Glu Phe Lys Cys Lys Val Asn Asn Lys Asp Leu
                325                 330                 335

Pro Ala Pro Ile Glu Arg Thr Ile Ser Lys Pro Lys Gly Ser Val Arg
            340                 345                 350

Ala Pro Gln Val Tyr Val Leu Pro Pro Pro Glu Glu Glu Met Thr Lys
        355                 360                 365

Lys Gln Val Thr Leu Thr Cys Met Val Thr Asp Phe Met Pro Glu Asp
    370                 375                 380

Ile Tyr Val Glu Trp Thr Asn Asn Gly Lys Thr Glu Leu Asn Tyr Lys
385                 390                 395                 400

Asn Thr Glu Pro Val Leu Asp Ser Asp Gly Ser Tyr Phe Met Tyr Ser
                405                 410                 415

Lys Leu Arg Val Glu Lys Lys Asn Trp Val Glu Arg Asn Ser Tyr Ser
            420                 425                 430

Cys Ser Val Val His Glu Gly Leu His Asn His His Thr Thr Lys Ser
        435                 440                 445

Phe Ser Arg Thr Pro Gly Lys
    450                 455


<210>    39
<211>    217
<212>    PRT
<213>    Artificial Sequence

<220>
<223>    A8 light chain


<400>    39
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
                20                  25                  30

Ser Val Thr Trp Tyr Gln Gln Leu Pro Gly Thr Ala Pro Lys Leu Leu
            35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ser Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>   40
<211>   444
<212>   PRT
<213>   Artificial Sequence

<220>
<223>   B8 heavy chain


<400>   40
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
                20                  25                  30

Tyr Met Ser Trp Val Arg Gln Ala Pro Gly Lys Gly Leu Glu Trp Val
            35                  40                  45

Ser Gly Ile Ser His Asp Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr

65                  70                  75                  80

Leu Gln Met Asn Ser Leu Arg Ala Glu Asp Thr Ala Val Tyr Tyr Cys
                85                  90                  95

Ala Arg His Trp Thr Thr Phe Asp Tyr Trp Gly Gln Gly Thr Leu Val
            100                 105                 110

Thr Val Ser Ser Thr Thr Ala Pro Ser Val Tyr Pro Leu Ala Pro Val
        115                 120                 125

Cys Gly Asp Thr Thr Gly Ser Ser Val Thr Leu Gly Cys Leu Val Lys
    130                 135                 140

Gly Tyr Phe Pro Glu Pro Val Thr Leu Thr Trp Asn Ser Gly Ser Leu
145                 150                 155                 160

Ser Ser Gly Val His Thr Phe Pro Ala Val Leu Gln Ser Asp Leu Tyr
                165                 170                 175

Thr Leu Ser Ser Ser Val Thr Val Thr Ser Ser Thr Trp Pro Ser Gln
            180                 185                 190

Ser Ile Thr Cys Asn Val Ala His Pro Ala Ser Ser Thr Lys Val Asp
        195                 200                 205

Lys Lys Ile Glu Pro Arg Gly Pro Thr Ile Lys Pro Cys Pro Pro Cys
    210                 215                 220

Lys Cys Pro Ala Pro Asn Leu Leu Gly Gly Pro Ser Val Phe Ile Phe
225                 230                 235                 240

Pro Pro Lys Ile Lys Asp Val Leu Met Ile Ser Leu Ser Pro Ile Val
                245                 250                 255

Thr Cys Val Val Val Asp Val Ser Glu Asp Asp Pro Asp Val Gln Ile
            260                 265                 270

Ser Trp Phe Val Asn Asn Val Glu Val His Thr Ala Gln Thr Gln Thr
        275                 280                 285

His Arg Glu Asp Tyr Asn Ser Thr Leu Arg Val Val Ser Ala Leu Pro
    290                 295                 300

Ile Gln His Gln Asp Trp Met Ser Gly Lys Glu Phe Lys Cys Lys Val
305                 310                 315                 320

Asn Asn Lys Asp Leu Pro Ala Pro Ile Glu Arg Thr Ile Ser Lys Pro
                325                 330                 335

Lys Gly Ser Val Arg Ala Pro Gln Val Tyr Val Leu Pro Pro Pro Glu
            340                 345                 350

Glu Glu Met Thr Lys Lys Gln Val Thr Leu Thr Cys Met Val Thr Asp
        355                 360                 365

Phe Met Pro Glu Asp Ile Tyr Val Glu Trp Thr Asn Asn Gly Lys Thr
    370                 375                 380

Glu Leu Asn Tyr Lys Asn Thr Glu Pro Val Leu Asp Ser Asp Gly Ser
385                 390                 395                 400

Tyr Phe Met Tyr Ser Lys Leu Arg Val Glu Lys Lys Asn Trp Val Glu

405                 410                 415

Arg Asn Ser Tyr Ser Cys Ser Val Val His Glu Gly Leu His Asn His
            420                 425                 430

His Thr Thr Lys Ser Phe Ser Arg Thr Pro Gly Lys
        435                 440

<210>   41
<211>   217
<212>   PRT
<213>   Artificial Sequence

<220>
<223>   B8 light chain

<400>   41
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Thr Trp Tyr Gln Gln Leu Pro Gly Thr Ala Pro Lys Leu Leu
        35                  40                  45

Ile Tyr Ala Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Tyr Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210>   42

<211>    455
<212>    PRT
<213>    Artificial Sequence

<220>
<223>    D9 heavy chain


<400>    42
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Tyr Pro Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ile Leu Pro Cys Pro Trp Gly Arg Cys Tyr Tyr Asp Tyr
            100                 105                 110

Ala Met Asp Val Trp Gly Gln Gly Thr Leu Val Thr Val Ser Ser Thr
        115                 120                 125

Thr Ala Pro Ser Val Tyr Pro Leu Ala Pro Val Cys Gly Asp Thr Thr
    130                 135                 140

Gly Ser Ser Val Thr Leu Gly Cys Leu Val Lys Gly Tyr Phe Pro Glu
145                 150                 155                 160

Pro Val Thr Leu Thr Trp Asn Ser Gly Ser Leu Ser Ser Gly Val His
                165                 170                 175

Thr Phe Pro Ala Val Leu Gln Ser Asp Leu Tyr Thr Leu Ser Ser Ser
            180                 185                 190

Val Thr Val Thr Ser Ser Thr Trp Pro Ser Gln Ser Ile Thr Cys Asn
        195                 200                 205

Val Ala His Pro Ala Ser Ser Thr Lys Val Asp Lys Lys Ile Glu Pro
    210                 215                 220

Arg Gly Pro Thr Ile Lys Pro Cys Pro Pro Cys Lys Cys Pro Ala Pro
225                 230                 235                 240

Asn Leu Leu Gly Gly Pro Ser Val Phe Ile Phe Pro Pro Lys Ile Lys
                245                 250                 255

Asp Val Leu Met Ile Ser Leu Ser Pro Ile Val Thr Cys Val Val Val
            260                 265                 270

Asp Val Ser Glu Asp Asp Pro Asp Val Gln Ile Ser Trp Phe Val Asn
        275                 280                 285

Asn Val Glu Val His Thr Ala Gln Thr Gln Thr His Arg Glu Asp Tyr
    290                 295                 300

Asn Ser Thr Leu Arg Val Val Ser Ala Leu Pro Ile Gln His Gln Asp
305                 310                 315                 320

Trp Met Ser Gly Lys Glu Phe Lys Cys Lys Val Asn Asn Lys Asp Leu
                325                 330                 335

Pro Ala Pro Ile Glu Arg Thr Ile Ser Lys Pro Lys Gly Ser Val Arg
            340                 345                 350

Ala Pro Gln Val Tyr Val Leu Pro Pro Pro Glu Glu Glu Met Thr Lys
        355                 360                 365

Lys Gln Val Thr Leu Thr Cys Met Val Thr Asp Phe Met Pro Glu Asp
    370                 375                 380

Ile Tyr Val Glu Trp Thr Asn Asn Gly Lys Thr Glu Leu Asn Tyr Lys
385                 390                 395                 400

Asn Thr Glu Pro Val Leu Asp Ser Asp Gly Ser Tyr Phe Met Tyr Ser
                405                 410                 415

Lys Leu Arg Val Glu Lys Lys Asn Trp Val Glu Arg Asn Ser Tyr Ser
            420                 425                 430

Cys Ser Val Val His Glu Gly Leu His Asn His His Thr Thr Lys Ser
        435                 440                 445

Phe Ser Arg Thr Pro Gly Lys
    450                 455


<210>    43
<211>    217
<212>    PRT
<213>    Artificial Sequence

<220>
<223>    D9 light chain


<400>    43
Gln Ser Val Leu Thr Gln Pro Pro Ser Ala Ser Gly Thr Pro Gly Gln
1               5                   10                  15

Arg Val Thr Ile Ser Cys Ser Asp Ser Ser Ser Asn Ile Gly Ser Asn
            20                  25                  30

Thr Val Ser Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu Arg Thr

            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210> 44
<211> 455
<212> PRT
<213> Artificial Sequence

<220>
<223> H6 heavy chain

<400> 44
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser His Gly Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ile Ser Asn Cys His Leu Gly Val Cys Tyr Tyr Ser Asn
            100                 105                 110

Gly Met Asp Val Trp Gly Gln Gly Thr Leu Val Thr Val Ser Ser Thr
        115                 120                 125

Thr Ala Pro Ser Val Tyr Pro Leu Ala Pro Val Cys Gly Asp Thr Thr
    130                 135                 140

Gly Ser Ser Val Thr Leu Gly Cys Leu Val Lys Gly Tyr Phe Pro Glu
145                 150                 155                 160

Pro Val Thr Leu Thr Trp Asn Ser Gly Ser Leu Ser Ser Gly Val His
                165                 170                 175

Thr Phe Pro Ala Val Leu Gln Ser Asp Leu Tyr Thr Leu Ser Ser Ser
            180                 185                 190

Val Thr Val Thr Ser Ser Thr Trp Pro Ser Gln Ser Ile Thr Cys Asn
        195                 200                 205

Val Ala His Pro Ala Ser Ser Thr Lys Val Asp Lys Lys Ile Glu Pro
    210                 215                 220

Arg Gly Pro Thr Ile Lys Pro Cys Pro Pro Cys Lys Cys Pro Ala Pro
225                 230                 235                 240

Asn Leu Leu Gly Gly Pro Ser Val Phe Ile Phe Pro Pro Lys Ile Lys
                245                 250                 255

Asp Val Leu Met Ile Ser Leu Ser Pro Ile Val Thr Cys Val Val Val
            260                 265                 270

Asp Val Ser Glu Asp Asp Pro Asp Val Gln Ile Ser Trp Phe Val Asn
        275                 280                 285

Asn Val Glu Val His Thr Ala Gln Thr Gln Thr His Arg Glu Asp Tyr
    290                 295                 300

Asn Ser Thr Leu Arg Val Val Ser Ala Leu Pro Ile Gln His Gln Asp
305                 310                 315                 320

Trp Met Ser Gly Lys Glu Phe Lys Cys Lys Val Asn Asn Lys Asp Leu
                325                 330                 335

Pro Ala Pro Ile Glu Arg Thr Ile Ser Lys Pro Lys Gly Ser Val Arg
            340                 345                 350

Ala Pro Gln Val Tyr Val Leu Pro Pro Pro Glu Glu Glu Met Thr Lys
        355                 360                 365

Lys Gln Val Thr Leu Thr Cys Met Val Thr Asp Phe Met Pro Glu Asp
    370                 375                 380

Ile Tyr Val Glu Trp Thr Asn Asn Gly Lys Thr Glu Leu Asn Tyr Lys
385                 390                 395                 400

Asn Thr Glu Pro Val Leu Asp Ser Asp Gly Ser Tyr Phe Met Tyr Ser
                405                 410                 415

Lys Leu Arg Val Glu Lys Lys Asn Trp Val Glu Arg Asn Ser Tyr Ser
            420                 425                 430

Cys Ser Val Val His Glu Gly Leu His Asn His His Thr Thr Lys Ser
        435                 440                 445

Phe Ser Arg Thr Pro Gly Lys
    450                 455


<210>    45
<211>    217
<212>    PRT
<213>    Artificial Sequence

<220>
<223>    H6 light chain

<400>    45
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Asp Val Tyr Trp Tyr Gln Gln Leu Pro Gly Thr Ala Pro Lys Leu Leu
        35                  40                  45

Ile Tyr Ser Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215